# EUROPEAN PATENT APPLICATION

(11) **EP 2 415 457 A1**
(43) Date of publication of application: **08.02.2012**
(21) Application number: 10758892.3
(22) Date of filing: 02.04.2010
(51) Int. Cl.: A61K 8/81, A61K 8/27, A61Q 1/12

(54) **SOLID POWDER COSMETIC AND METHOD FOR PRODUCING THE SAME**

(30) Priority: 03.04.2009 JP 2009091135
(71) Applicant: Shiseido Company, Ltd., Chuo-ku Tokyo 104-8010 (JP)
(72) Inventor: KANEKO Katsuyuki, Yokohama-shi Kanagawa 224-8558 (JP); KURAHASHI Takuma, Yokohama-shi Kanagawa 224-8558 (JP); KUSABA Kentaro, Yokohama-shi Kanagawa 224-8558 (JP); SONOYAMA Yuji, Yokohama-shi Kanagawa 224-8558 (JP)
(74) Representative: Henkel, Breuer & Partner
(86) International application number: PCT/JP2010/056083
(87) International publication number: WO 2010/114125

(57) **Abstract**

An object of the present invention is to provide a solid powder cosmetic having an excellent long-lasting makeup effect and the feeling of use.

A solid powder cosmetic comprising: spherical poly(meth)acrylate particles having pores in the interiors and at the surfaces and having an average particle size of 3 to 20 µm, a specific surface area of 80 to 180 m²/g, and a most frequent pore diameter of 180 Å or larger. Furthermore, it is suitable that the solid powder cosmetic further contains activated zinc oxide in an amount of 001% to 30% by mass based on the composition.

## Description

### RELATED APPLICATIONS

The present patent application claims priority of Japanese Patent Application No. 2009-091135 filed on April 3, 2009, the disclosure of which has been incorporated herein by reference.

### FIELD OF THE INVENTION

The present invention relates to a solid powder cosmetic and a method for producing the same, and more particularly, to an improvement of the long-lasting makeup effect and the feeling of use of a solid powder cosmetic.

### BACKGROUND OF THE INVENTION

Solid powder cosmetics such as foundation have been traditionally prepared by incorporating a powder having a sebum absorbing properties, for the purpose of suppressing makeup smudging, which occurs as a result of oiling up by sweat or sebum. Examples of such a powder that can be used include spherical powders of resins such as polymethyl methacrylate (PMMA) and a silicone resin. These powders prevent shining of the skin by utilizing scattering of light at the spherical surface, in addition to the sebum absorbing properties of the particles themselves, and thus, the powders are expected to give an effect of optically suppressing makeup smudging. Furthermore, the powders also have an excellent feeling of use such as a feeling of fit to the skin, owing to their morphological characteristics, and a finish with a good long-lasting makeup effect can be obtained.
However, these conventional resin powders are such that the sebum absorbing properties of the powders themselves are not that high, and even if the additional effects based on the optical and morphological characteristics described above are considered, it has been difficult to obtain a sufficient long-lasting makeup effect with the powders by themselves.

Thus, attempts have been made to (1) make the resin powders porous, and (2) use powders having different sebum absorbing properties in combination, in order to obtain a superior long-lasting makeup effect.
Examples of technologies related to item (1) include those disclosed in Japanese patent unexamined publication (JP-A)No. S57-98205, No. 2003-81738, and No. 2006-117920. That is, JP-A No. S57-98205 describes that excellent makeup sustainability was obtained by a makeup cosmetic produced by incorporating a spherical porous resin powder having a particle size of 1 to 40 µm and an average particle size of 2 to 20 µm. JP-A No. 2003-81738 describes that a skin cosmetic containing 3% to 30% by mass of spherical porous particles of a crosslinked polymer having a crosslinking density of 3% to 15% by mass, an average particle size of 3 to 15 µm, and a surface area of 5 to 50 m²/g, prevented makeup smudging and sustained a feeling of freshness. Furthermore, JP-A No. 2006-117920 describes that in the production of polymer particles having pores in the interior, bleeding of surfactants that cause degeneration of products incorporated with the polymer particles, such as cosmetics, was reduced.

However, in the porous resin powders, the effect of the particle size and the specific surface area which depends on the diameter or density of the pores on the particle surfaces, on the sebum absorbing properties of the powder is very large, and this also applies to the additional effects offered by the optical and morphological characteristics. That is, a powder which is presumed to have very fine pore diameters relative to the particle size and to have, therefore, a large specific surface area, such as described in JP-A No. S57-98205, has an increased sebum absorption capacity as compared with a poreless powder. However, since the pores are so small that the rate of sebum absorption is slow, and swelling of the particles due to sebum absorption is also not recognized, the overall sebum absorption efficiency of the powder is very low. In addition, since the pores are small, there was almost no chance of expecting an enhancement of the light scattering effect caused by the concavity and convexity of the pores at the particle surfaces.
As in the case of the powder described in JP-A No. 2003-81738, when the specific surface area is smaller with respect to the particle size or the pore diameter, adjustment of the feeling of use by means of the particle size can be made possible. However, since the distribution of pores is small, the sebum absorption capacity of the particles themselves was very low, and the light scattering effect due to the concavity and convexity of the pores could not be sufficiently obtained.
The technology of JP-A No. 2006-117920 has succeeded in obtaining a powder with reduced bleeding of surfactants. However, the powder is considered to be mainly used in molding materials or coating materials, and therefore, investigations on the powder conditions that are appropriate for the application to solid powder cosmetics, particularly sebum absorbing properties, were not made.
Therefore, a porous resin powder which is comprehensively excellent particularly in connection with the long-lasting makeup effect of solid powder cosmetics, is not obtainable for the present.

Furthermore, as a technology related to item (2), for example, JP-A No. S62-56415 discloses a cosmetic containing activated zinc oxide. In regard to this technology, the activated zinc oxide is incorporated as a sebum absorbing substance, as in the case of the spherical resin powder, and also, for the purpose of improving the feeling of use, a powder produced by coating spherical resin particles with activated zinc oxide is described.
However, the zinc oxide known as a sebum absorbing substance as described above acts only on fatty acids, which constitute only a very small portion of the sebum component, and does not act on triglycerides and the like that constitute the major part. Therefore, a sufficient long-lasting makeup effect could not be obtained with activated zinc oxide alone. Also, in regard to the zinc oxide-coated spherical resin powder, an enhancement of the feeling of use caused by the shape of the base material can be expected, but a clearly synergistic effect on the long-lasting makeup effect due to compositization or combined use, cannot be obtained.

[Patent Literature 1] Japanese patent unexamined publication No. S57-98205
[Patent Literature 2] Japanese patent unexamined publication No. 2003-81738
[Patent Literature 3] Japanese patent unexamined publication No. 2006-117920
[Patent Literature 4] Japanese patent unexamined publication No. S62-56415

### DISCLOSURE OF THE INVENTION

### PROBLEM TO BE SOLVED BY THE INVENTION

The present invention was made in view of such problems to be solved, and it is an object of the present invention to provide a solid powder cosmetic having an excellent long-lasting makeup effect.

### MEANS TO SOLVE THE PROBLEM

In order to solve the problems described above, the inventors of the present invention conducted a thorough investigation. As a result, the inventors found that when spherical poly(meth)acrylate particles having a specific particle size and a specific surface area are incorporated into a solid powder cosmetic, cosmetic smudging which is exhibited by shining of the skin is extremely reduced, and the condition immediately after makeup is put on can be maintained for a long time, thus completing the present invention.
Furthermore, the inventors found that when activated zinc oxide is further incorporated into the cosmetic, various sebum components that could not be completely absorbed when spherical poly(meth)acrylate particles or activated zinc is used alone, are removed, and the long-lasting makeup effect can be sustained for a long time.
The inventors also found that when a fluorine compound-treated powder is further incorporated into the cosmetic, the powder is imparted with water repellency and oil repellency, and also, dulling of color over time is suppressed, so that the long-lasting makeup effect is further improved.
The inventors also found that when the cosmetic is produced by using a facing rotor type mixing apparatus having a particular structure, various feelings of use such as a feeling of particulate fineness, a moist feeling and smoothness are excellent, and also, impact resistance is markedly improved.

That is, the solid powder cosmetic according to the present invention comprises spherical poly(meth)acrylate particles having pores in the interiors and at the surfaces and having an average particle size of 3 to 20 µm, a specific surface area of 80 to 180 m²/g, and a most frequent pore diameter of 180 Å or larger.
It is suitable for the solid powder cosmetic that the amount of incorporation of the spherical poly(meth)acrylate particles is 1% to 20% by mass based on the cosmetic.
It is also suitable for the solid powder cosmetic that the spherical poly(meth)acrylate particles are spherical polymethyl methacrylate (PMMA) particles.
Furthermore, it is suitable that the solid powder cosmetic further comprises an activated zinc oxide in an amount of 0.01 % to 30% by mass based on the cosmetic.

It is suitable that the solid powder cosmetic further comprises a fluorine compound-treated powder in an amount of 5% to 97% by mass based on the cosmetic.
It is also suitable for the solid powder cosmetic that the fluorine compound is 1H, 1H,2H,2H-perfluorooctyltriethoxysilane.

Furthermore, the method for producing a solid powder cosmetic according to the present invention comprises mixing of a powder component containing spherical poly(meth)acrylate particles which have pores in the interiors and at the surfaces, and has an average particle size of 3 to 20 µm, a specific surface area of 80 to 180 m²/g, and a most frequent pore diameter of 180 Å or larger, with an oily component, by using a facing rotor type mixing apparatus which has a first rotor provided with plural blades and a second rotor provided with plural blades, disposed in a mixing chamber such that the first rotor and the second rotor face each other and respectively have individual rotating shafts on the same axis line in an approximately horizontal direction, and which mixes raw materials by rotating the first rotor and the second rotor in the same or opposite direction to each other, while supplying the raw materials through a feed port on the first rotor side, and discharges the mixed raw materials through a discharge port on the second rotor side.
Furthermore, in the method for producing a solid powder cosmetic, it is suitable that the first rotor and the second rotor of the facing rotor type mixing apparatus rotate in the opposite direction to each other.

### EFFECT OF THE INVENTION

According to the present invention, there is obtained a solid powder cosmetic having an excellent long-lasting makeup effect acheived by incorporating spherical poly(meth)acrylate particles which have high sebum absorbing ability due to the polymer structure and pores, and are capable of suppressing greasy shining through a light scattering effect by maintaining the spherical form even after swelling as a result of the absorption of a large amount of sebum. Furthermore, the solid powder cosmetic can have the above-described effect further enhanced by the addition of activated zinc oxide, and is also advantageous in terms of simplification of the production process for the cosmetic, or production cost. The solid powder cosmetic is imparted with water repellency and oil repellency when a fluorine compound-treated powder is further incorporated therein, and also, dulling of color over time is suppressed so that the long-lasting makeup effect can be further improved. Furthermore, when the solid powder cosmetic is produced by using a facing rotor type mixing apparatus having a particular structure, a cosmetic which is excellent in various feelings of use such as a feeling of particulate fineness, a moist feeling, and smoothness, and also has a markedly improved impact resistance.

### BRIEF DESCRIPTION OF THE DRAWINGS

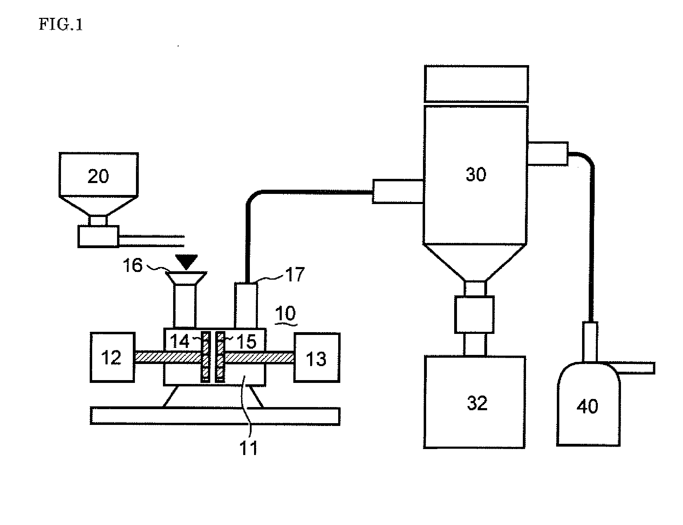

FIG. 1 is a schematic diagram showing an example of the facing rotor type mixing apparatus used in the production method of the present invention.

### DESCRIPTION OF REFERENCE NUMBERS

- 10: FACING ROTOR TYPE MIXING APPARATUS
- 11: MIXING CHAMBER
- 12: MOTOR
- 13: MOTOR
- 14: FIRST ROTOR
- 15: SECOND ROTOR
- 16: FEED PORT
- 17: DISCHARGE PORT
- 20: RAW MATERIAL SUPPLYING DEVICE
- 30: CAPTURING DEVICE
- 32: COLLECTING CONTAINER
- 40: SUCTION DEVICE

### BEST MODE FOR CARRYING OUT THE INVENTION

Hereinafter, the present invention will be described in detail.
The solid powder cosmetic according to the present invention contains spherical poly(meth)acrylate particles.

### <Spherical poly(meth)acrylate particles>

The spherical poly(meth)acrylate particles used in the present invention are composed of spherical polymer particles having plural pores in the interiors and at the surfaces. The spherical poly(meth)acrylate particles are obtained by radical polymerizing a monomer mixture containing one or more selected from (meth)acrylate ester-based monomers in the presence of a polymerization initiator and a porosifying agent. The particles can be produced by a general method for synthesizing spherical polymer particles, such as suspension polymerization, emulsion polymerization, or soap-free polymerization, but according to the present invention, it is particularly preferable that the particles be produced by a suspension polymerization method that will be described below.
The spherical poly(meth)acrylate particles used in the present invention can be produced by using water and a monomer phase mixture containing at least one monomer selected from acrylate ester-based monomers and methacrylate ester-based monomers (hereinafter, collectively referred to as "(meth)acrylate ester-based monomers"), by a known suspension polymerization method.

The monomer phase mixture contains a (meth)acrylate-based monomer which is polymerized while water is dispersed among the monomer molecules and turns into polymer particles having plural pores in the interiors and at the surfaces, a polymerization initiator which accelerates polymerization of the (meth)acrylate-based monomer, and a comb-like polymer which disperses the water among the (meth)acrylate ester-based monomer molecules.

Examples of the (meth)acrylate ester-based monomer include α-methylene aliphatic monocarboxylic acid esters such as methyl acrylate, ethyl acrylate, n-butyl acrylate, isobutyl acrylate, propyl acrylate, n-octyl acrylate, dodecyl acrylate, 2-ethylhexyl acrylate, stearyl acrylate, 2-chloroethyl acrylate, phenyl acrylate, methyl α-chloroacrylate, methyl methacrylate, ethyl methacrylate, n-butyl methacrylate, isobutyl methacrylate, propyl methacrylate, n-octyl methacrylate, dodecyl methacrylate, 2-ethylhexyl methacrylate, stearyl methacrylate, 2-chloroethyl methacrylate, phenyl methacrylate, dimethylaminoethyl acrylate, dimethylaminoethyl methacrylate, diethylaminoethyl acrylate, and diethylaminoethyl methacrylate; (meth)acrylate derivatives such as acrylonitrile, methacrylonitrile, acrylamide, methacrylamide, 2-hydroxyethyl acrylate, 2-hydroxypropyl acrylate, 2-hydroxyethyl methacrylate, and 2-hydroxypropyl methacrylate; acrylic acid, methacrylic acid, and fumaric acid. These (meth)acrylate ester-based monomers can be used singly or as mixtures of two or more kinds. According to the present invention, it is particularly preferable to use methyl methacrylate as a monomer to obtain spherical polymethyl methacrylate (PMMA).

Furthermore, as the polymerization initiator that accelerates polymerization of the (meth)acrylate ester-based monomer, a polymerization initiator that is generally used for suspension polymerization of a (meth)acrylate ester-based monomer can be used. Examples of the polymerization initiator include peroxide-based polymerization initiators such as benzoyl peroxide, lauroyl peroxide, octanoyl peroxide, benzoyl ortho-chloroperoxide, benzoyl ortho-methoxyperoxide, methyl ethyl ketone peroxide, diisopropyl peroxydicarbonate, cumene hydroperoxide, cyclohexanone peroxide, t-butyl hydroperoxide, and diisopropylbenzene hydroperoxide; 2,2'-azobisisobutyronitrile, 2,2'-azobis(2,4-dimethylvaleronitrile), 2,2'-azobis(2-methylbutyronitrile), 2,2'-azobis(2,3,3-trimethylbutyronitrile)-2,2'-azobis(2-isopropylbutyronitrile), 11'-azobis(cyclohexane-1-carbonitrile), 2,2'-azobis(4-methoxy-2,4-dimethylvaleronitrile), 2-(carbamoylazo)isobutyronitrile, 4,4'-azobis(4-cyanovaleric acid), and dimethyl 2,2'-azobisisobutyrate. These polymerization initiators can be used singly or as mixtures of two or more kinds.
Benzoyl peroxide, lauroyl peroxide, 2,2'-azobisisobutyronitrile, and 2,2'-azobis(2,4-dimethylvaleronitrile) are suitable from the viewpoint that these compounds are easily dissolved in the monomer, and handling thereof is easy.
The amount of incorporation of the polymerization initiator varies depending on the monomer used, but is usually 0.01 to 1.00 parts by mass relative to 100 parts by mass of the monomer.

The comb-like polymer has many trifurcation points with linear side chains in the main chain, and usually has a weight average molecule of 2000 to 100000.
The comb-like polymer is not particularly limited as long as the polymer has a hydrophilic portion and a hydrophobic portion in the molecule. However, from the viewpoint that the water dispersed among the monomer molecules can be easily stabilized in a particulate form, a comb-like polymer in which plural side chains constituting the hydrophobic portion are graft-bonded to the main chain having the hydrophilic portion is suitable. Examples of the comb-like polymer that can be used include a poly(ethyleneimine) which has one or more poly(carbonyl-C3-C6 alkyleneoxy) chain, while each chain has three to eighty carbonyl-C3-C6 alkyleneoxy groups and is bonded to poly(ethyleneimine) through an amide or salt-crosslinking group; an acid salt of such a polyethyleneimine; and a reaction product between a poly(lower alkylene)imine and a polyester having a free carboxylic acid group, in which at least two or more polyester chains are bonded to each poly(lower alkylene)imine chain. As such a comb-like polymer, for example, the "SOLSPERSE" series that are commercially available from Lubrizol Corp. in the United Kingdom, can be used.

The comb-like polymer material preferably has an acid value of 20 to 80. If the acid value is less than 20, the polymer particles may not all have plural pores formed in the interiors and at the surfaces of the polymer particles, and if the acid value is greater than 80, polymerization becomes unstable, and the polymer may not be obtained as a particulate material. The acid value can be measured as the number of milligrams of KOH required to neutralize free carboxylic acids contained in 1 g of the comb-like polymer, according to JIS K0070.

The amount of incorporation of the comb-like polymer material is suitably 0.01 to 3.00 parts by mass relative to 100 parts by mass of the monomer. If the amount of incorporation is less than 0.01 parts by mass, the polymer particles may not all have plural pores in the interiors and at the surfaces. On the other hand, if the comb-like polymer is incorporated in an amount of even greater than 3 parts by mass, a pore forming effect (ease of forming pores) adequate for the amount of incorporation may not be obtained, while there is a risk that the comb-like polymer material may decrease the purity of the polymer and impair the properties of the polymer.

Furthermore, in addition to the components described above, a crosslinking agent for the monomer, another monomer or oligomer, and the like can be added to the monomer phase mixture, to the extent that the effect of the invention is not impaired.
Such a crosslinking agent may be a compound having two or more polymerizable double bonds, and examples include aromatic divinyl compounds such as divinylbenzene, divinylnaphthalene, and derivatives thereof; diethylenic carboxylic acid esters such as ethylene glycol dimethacrylate, triethylene glycol triacrylate, and trimethylolpropane triacrylate; and compounds having three or more divinyl compounds and vinyl groups, such as N,N-divinylaniline, divinyl ether, and divinyl sulfite. These compounds can be used singly or as mixtures of two or more kinds.
Furthermore, examples of the other monomer or oligomer include styrene and derivatives thereof, and vinyl esters such as vinyl butyrate.

Into the water which disperse the monomer phase mixture, adispersion stabilizer or a surfactant for aqueous media can be incorporated.
As the dispersion stabilizer, a dispersion stabilizer that is generally used in suspension polymerization of polymers can be used, and examples that can be used include water-soluble polymers such as methyl cellulose, hydroxyethyl cellulose, and polyvinyl alcohol; and sparingly water-soluble inorganic salts such as tribasic calcium phosphate, magnesium hydroxide, magnesium pyrophosphate, barium sulfate, calcium carbonate, and silica. Particularly, from the viewpoint of being easily removable from the polymer after polymerization and being capable of polymerizing polymer particles with a narrow particle size distribution, a sparingly water-soluble inorganic salt having a solubility in water at normal temperature of about 3 mg or less, and tribasic calcium phosphate having a solubility of 2.5 mg is suitable.
The dispersion stabilizer is usually incorporated into water at a proportion of 0.1 to 20.0 parts by mass relative to 100 parts by mass of the polymer particles that are obtained.

As the surfactant for aqueous media, a surfactant for aqueous media that is generally used in suspension polymerization of polymers can be used. From the viewpoint that polymer particles can be polymerized with a narrow particle size distribution, anionic surfactants such as sodium dodecyl sulfate, sodium dodecyl benzenesulfonate, sodium polyoxyethylene lauryl ether sulfate, and sodium diethylsulfosuccinate are particularly preferred, and such a surfactant for aqueous media is incorporated into water at a concentration of 0.005% to 0.3% by mass.

Next, a specific method for producing spherical poly(meth)acrylate particles will be described.
The monomer phase mixture and water are respectively adjusted to predetermined amounts of incorporation in separate vessels and are mixed. That is, on one side, a (meth)acrylate-based monomer, a comb-like polymer, a polymerization initiator, a crosslinking agent, another monomer, an oligomer and the like are mixed and agitated at predetermined proportions as a monomer phase mixture. As a mixing and agitating unit that may be used at this time, a general mixer or a homogenizer can be used, but it is preferable to employ a mixing and agitating unit that would make the system thoroughly uniform. Furthermore, when there is a risk that the temperature of the monomer phase mixture may increase as a result of mixing and agitation, and polymerization of the (meth)acrylate ester-based monomer may be initiated, it is preferable to mix and agitate the system while suppressing the temperature increase using a cooling unit or the like.

On the other side, as a material for aqueous phase, a dispersion stabilizer and a surfactant for aqueous media are added to water at predetermined proportions, and the mixture is mixed and stirred. In this case as well, a general mixer or a homogenizer can be used as a mixing and agitating unit to be used, and it is preferable to employ a mixing and agitating unit that would make the system thoroughly uniform.

Thereafter, the monomer phase mixture is poured into the water that has been prepared as described above, and the resulting mixture is mixed and agitated with a homogenizer or the like, to obtain a suspension liquid (aqueous phase/monomer phase/aqueous phase emulsion). At this time, the particle size of the monomer phase, that is, the particle size of the polymer particles having plural pores in the interiors and at the surfaces, can be easily adjusted by varying the conditions for agitation such as the time of agitation and the speed of rotation using a homogenizer as an agitating unit.
In regard to the production of the spherical poly(meth)acrylate particles according to the present invention, the particle size is adjusted to have an average particle size of 3 to 20 µm. If the average particle size is less than 3 µm, when the spherical poly(meth)acrylate particles are incorporated into a cosmetic, sufficient sebum absorbing properties are not exhibited, and an excellent long-lasting makeup effect cannot be obtained. If the average particle size is greater than 20 µm, the feeling of use of the cosmetic to which the spherical poly(meth)acrylate particles are incorporated tends to be deteriorated.

The suspension liquid is introduced into a heating apparatus such as an autoclave, and the suspension liquid is heated under stirring to thereby carry out polymerization of the monomer phase. The polymerization product thus obtained is filtered, and the filter cake is washed with water and dried. Thus, spherical poly(meth)acrylate particles having plural pores in the interiors and at the surfaces can be obtained.
Furthermore, if necessary, a process of removing the dispersion stabilizer before the washing can also be carried out.

In the spherical poly(meth)acrylate particles that may be obtained as described above, pores generated from a comb-like polymer that is finely dispersed in the monomer phase by mixing as a template, are formed in a large number in the interiors and at the surfaces.
In the production of the spherical poly(meth)acrylate particles according to the present invention, a known material which is known as a porogen can be used, in addition to the comb-like polymer described above, as the material that forms plural pores (porosification) in the interiors and at the surfaces of the polymer particles. Examples of the porogen include toluene, isooctane, methyl isobutyl ketone, calcium carbonate, tricalcium phosphate, and various linear polymers. These compounds can be used singly or in combination of two or more kinds. Definitely, the method for producing spherical poly(meth)acrylate particles can be appropriately modified in accordance with the porosifying material used, regardless of the method described above.

According to the present invention, the pore diameter or shape of the pores in the interiors and at the surfaces of the spherical poly(meth)acrylale particles, and the specific surface area of the particles can be regulated by known techniques in accordance with the type of the porosifying material. That is, the pore diameter of the pores and the specific surface area of the spherical poly(meth)acrylate particles can be regulated by appropriately selecting the method for application of the porosifying material to the polymer particle synthesis step (including the conditions for agitation), the amount of application or the like, in accordance with the characteristics of the material used.
According to the present invention, particularly, use is made of spherical poly(meth)acrylate particles formed such that the specific surface area of the particles is 80 to 180 m²/g.
If the specific surface area is less than 80 m²/g, the effects of sebum absorption and light scattering by the pores cannot be sufficiently obtained. Furthermore, if the specific surface area is larger than 180 m²/m, the pores are so densely present that the sebum absorption capacity of the polymer particles themselves is decreased, and also, the feeling of fit to the skin by the particles is lost. Thus, the finish of makeup is deteriorated.

For the spherical poly(meth)acrylate particles according to the present invention, the most frequent pore diameter of the pores in the interiors and at the surfaces of the particles is preferably 180 Å or greater. When the most frequent pore diameter is 180 Å or greater, the oil absorption (oleic acid, which is a sebum component) of the particles reaches 100 to 300 ml/100 g, and makeup smudging due to sebum can be prevented to a large extent. If the most frequent pore diameter is less than 180 Å, since the pores are small, the oil absorption efficiency and the oil absorption are decreased, and a sufficient long-lasting makeup effect may not be obtained.
The most frequent pore diameter described above has no particular upper limit in achieving the long-lasting makeup effect of the present invention. However, if the strength of the particles, the feeling of use at the time of being incorporated into a cosmetic, and the like are to be considered, the most frequent pore diameter is preferably adjusted to about 180 to 400 Å.

According to the present invention, commercially available products may also be used as the spherical poly(meth)acrylate particles having pores in the interiors and at the surfaces, as long as the powder has the particle size and the specific surface area described above. An example of such a powder that can be suitably used may be "TECHPOLYMER MBP-8HP" manufactured by Sekisui Chemical Co., Ltd.

The amount of incorporation of the spherical poly(meth)acrylate particles in the solid powder cosmetic according to the present invention is preferably 1% to 20% by mass based on the cosmetic composition. If the amount of incorporation is less than 1% by mass, the spherical poly(meth)acrylate particles exhibit insufficient sebum absorption, and the spherical particles do not give a feeling of use with an excellent feeling of fit. If the amount of incorporation is greater than 20% by mass, a sebum absorption effect is obtained, but friction or slipperiness occurs at the time of applying the cosmetic, so that the feeling of use or the beauty of the finish is deteriorated. Furthermore, the amount of incorporation of the spherical poly(meth)acrylale particles is more preferably 1% to 10% by mass.

### <Activated zinc oxide>

It is also preferable to further incorporate activated zinc oxide to the solid powder cosmetic according to the present invention, in addition to the spherical poly(meth)acrylate particles, from the viewpoint of further increasing the long-lasting makeup effect.
The activated zinc oxide as used herein refers to zinc oxide that is produced by a wet method, and is obtained by, for example, allowing a zinc sulfate (zinc oxide) solution and a soda ash solution to react, calcining the reaction product, and pulverizing the resultant product. Such activated zinc oxide has a larger specific surface area as compared with the zinc oxide that is obtained by a dry method and is used in the conventional cosmetic field. The specific surface area of conventional zinc oxide is generally less than 15 m²/g, but the specific surface area of the activated zinc oxide according to the present invention is usually 25 m²/g or greater.

The activated zinc oxide produced as described above may have some residual basic zinc carbonate, depending on the conditions for calcination. If the amount of the residual basic zinc carbonate is too large, the long-lasting makeup effect of the solid powder cosmetic to which the activated zinc oxide has been incorporated, may not be sufficiently exhibited. Therefore, in regard to the incorporation into the solid powder cosmetic according to the present invention, it is preferable to use an activated zinc oxide having an amount in terms of zinc oxide (JIS K1410) of 75% to 100% by mass.
The amount of incorporation of the activated zinc oxide in the solid powder cosmetic according to the present invention is 0.01% to 30% by mass based on the cosmetic composition.

The components constituting sebum are known to include triglycerides, diglycerides, monoglycerides, free fatty acids, squalene and wax esters, which are sebaceous gland-derived lipids; and cholesterol and cholesterol esters, which are keratinocyte-derived lipids.
The activated zinc oxide absorbs free fatty acids, which occupy about 16% by mass of the sebum components, particularly efficiently. However, the activated zinc oxide almost does not absorb triglycerides, which are principal components occupying about 40% by mass of the sebum components.
On the other hand, the spherical poly(meth)acrylate particles having a particular structure according to the present invention as described above have remarkably excellent properties for absorbing triglycerides that are particularly on the skin, as compared with the conventional spherical polymer particles.
Therefore, when the spherical poly(meth)acrylate particles and activated zinc oxide are incorporated together in appropriate amounts into the solid powder cosmetic of the present invention, every component of sebum is efficiently absorbed by the respective powders, and the long-lasting makeup effect can be realized over a longer time period.

### <Fluorine compound-treated powder>

The powder components used in the present invention include the spherical poly(meth)acrylate powder (and activated zinc oxide), and powder components that are not surface-treated may be used, or powder components that are surface-treated with silicones, fluorine compounds, fatty acid soaps and the like may also be used. However, it is particularly preferable to incorporate powder components that are surface-treated with fluorine compounds.
When a fluorine compound-treated powder component is incorporated into the solid powder cosmetic, water repellency and oil repellency are imparted to the solid powder cosmetic, and the solid powder cosmetic does not easily dissolve in sweat or sebum. Also, dulling of color over time does not easily occur.

Examples of the fluorine compound that is used to treat the powder surface include perfluoroalkyl phosphoric acid ester-diethanolamine salts, perfluoroalkylsilanes, perfluoroalkyl ethyl acrylates; and compounds having perfluoropolyether groups, such as perfluoropolyether dialkyl phosphoric acid and salts thereof, perfluoropolyether dialkyl sulfates and salts thereof, perfluoropolyether dialkyl carboxylic acid and salts thereof. Particularly, fluorine compounds having any one perfluoroalkyl group among CF₂-, CF₃-, CF₃CF₂-, and CF₂CF₂- in the molecule are suitable.

As the fluorine compound, 1H,1H,2H,2H-perfluorooctyltriethoxysilane represented by the following formula (I) can be particularly suitably used.

CF₃CF₂CF₂CF₂CF₂CF₂CH₂CH₂Si(OEt)₃ (I)

Furthermore, specific examples of other fluorine compounds include fluorine compounds represented by the following formulas (II) to (IV).

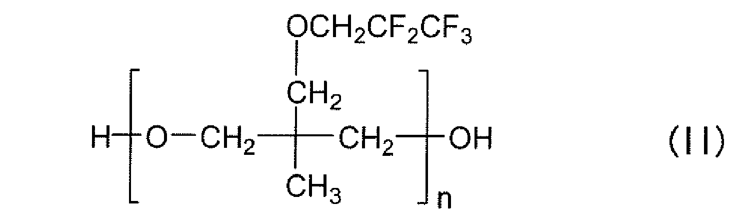

wherein in the formula (II), n represents an integer from 3 to 25.

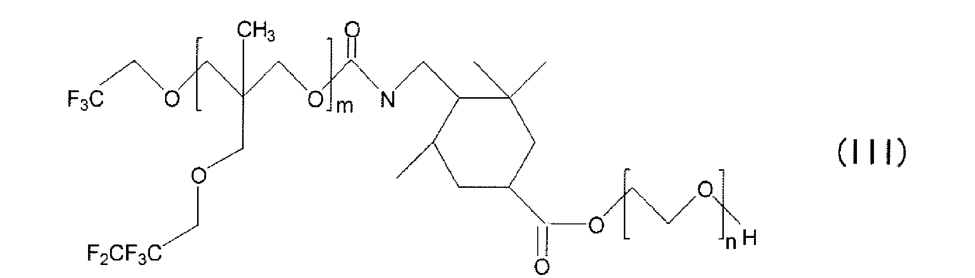

wherein in the formula (III), m and n each represent integer from 5 to 20.

X-CF₂O-(CF₂CF₂O)_{P}-(CF₂O)_{q}-CF₂-X (IV)

wherein in the formula (IV), X represents any one of CH₂OH, CO-NH-C₁₈H₃₇, and CH₂C-(OCH₂CH₂)p-OPO(OH)₂; and the p/q ratio represents an integer from 0.5 to 3.0.

Furthermore, a surface treatment of the powder components may be carried out by using the fluorine compound and another hydrophobization treating agent in combination. Specific examples of other treating agents include an acrylic silicone compound represented by the following formula (V). 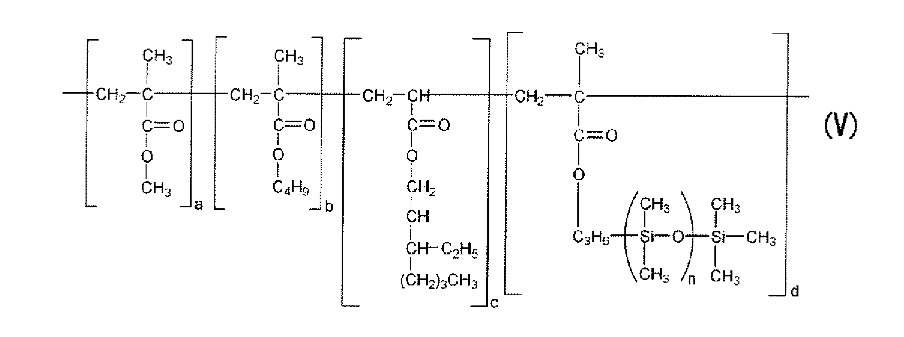 wherein n represents an integer; a, b, c and d represent the respective molar ratios in the copolymer, and are not necessarily zero; and d is from 40% by mole to 60% by mole.

The surface treatment of the powder component with a fluorine compound can be carried out according to a routine method.
For example, a surface-treated powder can be produced by bringing the fluorine compound of the formula (I) (and the acrylic silicone compound of the formula (V)) into contact with the powder while the compounds are in the form of a solution in which the fluorine compound is dissolved in an appropriate solvent, or in the form of the liquid of the fluorine compound itself, and then heating the resultant at 100°C to 150°C, and preferably 120°C to 140°C, for 1 to 12 hours, and preferably 3 to 9 hours.
For the heating atmosphere, the heating may be carried out in air, which is a moisture-containing atmosphere, or in another gas containing moisture at least to the extent that is contained in air. In addition to these, the heating may also be carried out by a method of producing the surface-treated powder in an atmosphere that does not contain moisture, and then heating the surface-treated powder while adding moisture during the treatment (during heating); or by a method of adding a solution containing one or more metal salts of aluminum (III), tin (II), tin (IV), iron (III) or titanium (III) in a small amount of moisture, simultaneously with or previously to the surface treating agent (the fluorine compound of formula (I) (and the acrylic silicone compound of formula (V)). Specific examples of the metal salts include aluminum chloride, stannic chloride, and ferric chloride (including hydrates thereof).

When the fluorine compound of the formula (I) (and the acrylic silicone compound of the formula (V)) is brought into contact with the powder in the form of a solution in which the fluorine compound is dissolved in an appropriate solvent, for example, a solution containing 0.3% to 50% by mass of the fluorine compound in a solvent such as an alcohol, water, hexane, cyclohexane or toluene is prepared. The powder is dispersed in the solution, and then the dispersion is heated. Thereby, the solvent is evaporated, and at the same time, the fluorine compound of the formula (I) (and the acrylic silicone compound of the formula (V)) is polymerized on the surfaces of the powder. Thus, a treated powder is obtained. This process can be carried out using a Henschel mixer, a Redige mixer, a kneader, a medium agitating mill (bead mill or the like), or the like. As the apparatus used in the heating, an electric furnace, a tunnel furnace, a roller hearth kiln, a rotary kiln, or the like.

When the fluorine compound of the formula (I) (and the acrylic silicone compound of the formula (V)) is directly brought into contact with the powder without dissolving the compound in a solvent, the fluorine compound is brought into contact with the powder using an appropriate mixing machine such as, for example, a rotary ball mill, a vibratory ball mill, a planetary ball mill, a sand mill, an attriter, a pug mill, a pony mixer, a planetary mixer, a Raikai mixer, a Henschel mixer, or the like. Thus, a treated powder is obtained.

The amount of incorporation of the fluorine compound-treated powder in the solid powder cosmetic of the present invention is preferably 5% to 97% by mass, and more preferably 20% to 75% by mass, relative to the total amount of the powder cosmetic. If the amount of incorporation of the fluorine compound-treated powder is less than 5% by mass, the effect of incorporating the fluorine compound-treated powder is not sufficiently obtained. On the other hand, if the amount of incorporation exceeds 97% by mass, the feeling of use may be deteriorated.

The solid powder cosmetic according to the present invention can be produced by a routine method, by incorporating, in addition to the constituent components described above, the components that are conventionally used in solid powder cosmetics to the extent that does not impair the effects of the present invention.
Examples of the components that are conventionally used in solid powder cosmetics include oil components, powders, ultraviolet protective agents, anionic surfactants, cationic surfactants, amphoteric surfactants, nonionic surfactants, moisturizing agents, water-soluble polymers, thickening agents, film-forming agents, metal ion sequestering agents, lower alcohols, polyhydric alcohols, pH adjusting agents, antioxidants, antioxidant aids, fragrances, and water. These components can be appropriately incorporated as necessary.

Particularly, the amount of incorporation of the oil components is preferably set to 5% to 40% by mass in total, and more suitably to 6% to 25% by mass in total, based on the cosmetic composition. If the amount of incorporation of the oil components is less than 5% by mass, it may be difficult to maintain the quality, such as being susceptible to an impact such as dropping, depending on the form of the product. If the amount of incorporation is greater than 40% by mass, the long-lasting makeup effect of the sebum absorbing powder described above may be deteriorated by the oil components in the cosmetic composition.

### <Method for producing solid powder cosmetic>

The solid powder cosmetic of the present invention can be produced by mixing various constituent components including the powder components and oily components explained above, according to a routine method. However, it is particularly preferable to produce the solid powder cosmetic by mixing the powder components and the oily components using a facing rotor type mixing apparatus having a particular structure that will be described below. When the facing rotor type mixing apparatus is used, the powder components can be uniformly coated on the surfaces with the oily components, without causing aggregation of the powder components. Thus, the solid powder cosmetic obtainable thereby is excellent in various feelings of use such as a feeling of particulate fineness, a moist feeling and smoothness, and also has a markedly improved impact resistance.

Here, the facing rotor type mixing apparatus used in the present invention is a mixing apparatus which has a first rotor provided with plural blades and a second rotor provided with plural blades, disposed in a mixing chamber such that the first rotor and the second rotor face each other and respectively have individual rotating shafts on the same axis line in an approximately horizontal direction, and which mixes raw materials by rotating the first rotor and the second rotor in the same or opposite direction to each other, while supplying the raw materials through a feed port on the first rotor side, and discharges the mixed raw materials through a discharge port on the second rotor side.

When the powder components and the oily components are mixed using such a facing rotor type mixing apparatus as described above, the powder particles can be uniformly coated on the surfaces with the oily components, without causing aggregation of the powder components. Furthermore, since the facing rotor type mixing apparatus used in the present invention is a dry type mixing apparatus, it is not necessary to use the powder components and the oily components after dissolving the components in an appropriate solvent for mixing, and the preparation process is simple and easy as compared with the case of wet mixing. Also, the facing rotor type mixing apparatus is less problematic in terms of safety or in terms of environment.

The facing rotor type mixing apparatus used in the present invention has been conventionally used as an apparatus for pulverization, and is well known to those having ordinary skill in the art as a pulverizing apparatus. For example, the pulverizing apparatuses described in JP-A Nos. 2002-79183, 2003-1127, 2003-10712, 2003-71307, and the like, can be used as the mixing apparatus of the present invention. In addition, examples of commercially available apparatuses include a cyclone mill (manufactured by Flo-tec, Ltd.).

A schematic diagram of one example of the facing rotor type mixing apparatus used in the present invention is shown in Fig. 1. However, the facing rotor type mixing apparatus used in the present invention is not limited to this.
In the facing rotor type mixing apparatus 10, a first rotor 14 and a second rotor 15, which are respectively driven to rotate by motors 12 and 13, are provided inside a mixing chamber 11, in which the first rotor and the second rotor face each other on the same axis line in the horizontal direction. The first rotor 14 and the second rotor 15 are provided to be in communication with a feed port 16 for the raw materials on the first rotor 14 side in the mixing chamber 11, and is in communication with a discharge port 17 on the second rotor 15 side in the mixing chamber 11. Furthermore, a raw material supplying device 20 is provided in the upper part of the feed port 16 of the facing rotor type mixing apparatus 10, and a capturing device 30 (and a collecting container 32) and a suction device 40 are connected to the end of the discharge port 17.

In the facing rotor type mixing appparatus 10, the first rotor 14 and the second rotor 15 that are disposed to face each other on the same axis line in the horizontal direction, rotate integrally with the rotating axes of the motors 12 and 13, respectively. In the facing rotor type mixing apparatus 10, while the first rotor 14 and the second rotor 15 are rotated by the motors 12 and 13 at high speed in the same or in the opposite direction to each other, a raw material mixture to be treated is introduced by the raw material supplying device 20 through the raw material feed port 16. The raw material mixture introduced to the facing rotor type mixing apparatus 10 vigorously collides with the first rotor 14, the second rotor 15, or the inner wall surfaces of the mixing chamber 11, and also, the molecules of the raw material components collide with each other, so that the raw material components are uniformly mixed and dispersed. As a result, there is obtained a mixture in which the powder particles are uniformly coated on the surfaces with the oily components, without causing aggregation of the powder components.

Furthermore, the first rotor 14 and the second rotor 15 that are facing each other, rotate in the same or opposite direction to each other. Here, in the production method of the present invention, it is suitable to use the first rotor and the second rotor to rotate in the opposite direction. When the rotors are rotated in the opposite direction to each other, a larger shear stress can be generated as compared with the case of rotating the rotors in the same direction. Therefore, a uniform mixture in which aggregation of the powder components does not easily occur, is likely to be obtained. Furthermore, the speed of rotation of the first rotor 14 and the second rotor 15 can be appropriately adjusted to be between, for example, 1000 rpm and 10000 rpm, and preferably between 3000 rpm and 8000 rpm.

In the first rotor 14 and the second rotor 15, plural blades are provided radially around the boss provided along the respective rotating shafts of the motors 12 and 13. Usually, the number of blades in one rotor is about 2 to 16 sheets. In the first rotor 14 and the second rotor 15, the shape of the rotor, the number of blades, and the like may be identical with or different from each other.

The subject mixture mixed inside the mixing chamber 11, is discharged through the discharge port 17. Furthermore, the capturing device 30 and the suction device 40 are connected to the end of the discharge port 17. When the suction device 40 is operated, the subject mixture is continuously discharged through the discharge port 17, and the subject mixture thus discharged is captured by the capturing device 30 and is collected inside the collecting container 32. The operating conditions for the suction device 40 can be appropriately adjusted through the type or amount of the subject mixture, the speed of rotation of the rotor, and the like. When the raw material mixture is continuously introduced by the raw material supplying device 20 while the suction device 40 and the capturing device 30 are operated, the mixture can be continuously produced.

The powder components and the oily components may be introduced to the facing rotor type mixing apparatus 10 separately or simultaneously. However, in typical cases, it is preferable to carry out preliminary mixing using a simple agitating apparatus such as a Henschel mixer or a Nauta mixer. When the powder components and the oily components are introduced to the facing rotor type mixing apparatus 10 without performing preliminary mixing, there are difficulties in the control of the mixing process, such as that only light powder components are first discharged without being sufficiently mixed with the oily components.

Furthermore, in the case of producing the solid powder cosmetic of the present invention, usually a mixture of the powder components and the oily components obtained as described above is filled in, for example, a middle-sized dish container made of a metal or a resin, and is solidified by dry molding. As the method for solidification, conventionally known dry press molding or the like can be used.

Furthermore, there are no particular limitations on the use of the solid powder cosmetic according to the present invention as a product, but the solid powder cosmetic can be applied to, for example, makeup base, foundation, cheek blush, eye shadow, eyeliner, suncare products, body cosmetic products, and the like.

### EXAMPLES

Hereinafter, the present invention will be described in more detail by way of Examples, but the present invention is not intended to be limited thereto. Unless particularly stated otherwise, the amount of incorporation is expressed in percent (%) by mass.

### <Porous spherical poly(meth)acrylate particles>

First, the method for producing the spherical poly(meth)acrylate particles used in the present Examples will be described.

### Production Example for spherical poly(meth)acrylate articles

Methyl methacrylate, ethylene glycol dimethacrylate, benzoyl peroxide, and a comb-like polymer (SOLSPERSE 26000, manufactured by Lubrizol Corp.) were mixed, and thus a monomer phase mixture was prepared. Furthermore, tribasic calcium phosphate, sodium dodecyl sulfate, and water were mixed to separately obtain an aqueous phase mixture.
The monomer phase mixture and the aqueous phase mixture thus obtained were mixed and agitated with a homomixer (3500 rpm), and a resulting suspension liquid was subjected to a polymerization reaction. Thus, spherical polymethyl methacrylate particles having pores having a most frequent pore diameter of 180 Å or greater in the interior and at the surfaces (spherical porous PMMA) were obtained.

Next, the service test and the evaluation method for the present Example will be described.

### Service test method

After washing the face, testers conditioned the facial skin with certain amounts of a skin toner and an emulsion, and then applied the foundations of various Test Examples on the face. The foundations were applied such that the foundations could be respectively compared, by applying the samples of other test examples on the right side and the left side of the face.
After the testers applied the foundations, the testers were allowed to stay in an artificial weather chamber adjusted to an air temperature of 32°C and a humidity of 65%, for 3 hours. Thus, the long-lasting makeup effect of the samples was evaluated according to the following criteria.

### Long-lasting makeup effect (oily shining)

Three expert evaluators carried out a visual evaluation of the oily shining of the skin after 3 hours of the application of the foundations, and scored from zero point (significant oily shining of the skin is recognized) to ten points (no oily shining of the skin is recognized). In this visual evaluation of the long-lasting makeup effect, the evaluators comprehensively evaluated the oily shining of the skin applied with the foundations.
Furthermore, the long-lasting makeup effect of each of the foundations was evaluated from the average point of the scores of the three evaluators (rounded at the first decimal place), according to the following criteria.
A: The average of the scores is 9 to 10 points.
B: The average of the scores is 6 to 8 points.
C: The average of the scores is 4 to 5 points.
D: The average of the scores is 2 to 3 points.
E: The average of the scores is 0 to 1 point.

### Feeling of use (feeling of mealiness)

A panel of thirty female testers scored the feeling of use of the respective foundations of the Test Examples according to the following criteria, and evaluated the average points (rounded at the first decimal place).
0: Very mealy, and markedly slippery.
1: Mealy and slippery.
2: Normal.
3: Smooth and fitting to the skin.
4: Very smooth, and fitting to the skin.

Foundations prepared by incorporating the spherical polymethyl methacrylate particles having pores in the interior and at the surfaces (spherical porous PMMA) obtained in the Production Example described above, and other sebum absorbing powders that are conventionally used (spherical crosslinked silicone, and spherical porous silica), by a conventional method, were evaluated for their long-lasting makeup effect. The results are presented in Table 1.

**[Table 1]**

| | Test Example | | | |
|---|---|---|---|---|
| | 1 | 2 | 3 | 4 |
| Silicone-treated talc | 20 | 20 | 20 | 20 |
| Silicone-treated sericite | Balance | Balance | Balance | Balance |
| Mica | 5 | 5 | 5 | 5 |
| Plate-shaped barium sulfate | 5 | 5 | 5 | 5 |
| Spherical porous PMMA | 10 | - | - | - |
| (particle size 10 µm, specific surface area 150 m²/g) | | | | |
| Spherical crosslinked silicone | - | 10 | - | 5 |
| (TREFIL E506C: manufactured by Dow Coming Toray Co., Ltd., average particle size 10 µm) | | | | |
| Spherical porous silica | - | - | 10 | 5 |
| (SUNSPHERE L51: manufactured by Asahi Glass Co., Ltd., average particle size 5 µm, pore diameter 130 Å, specific surface area 300 m²/g) | | | | |
| Silicone-treated titanium oxide | 10 | 10 | 10 | 10 |
| Alltylsilicone-treated iron oxide | 5 | 5 | 5 | 5 |
| Titanium oxide fine particles | 5 | 5 | 5 | 5 |
| Antiseptic | 0.2 | 0.2 | 0.2 | 0.2 |
| Diphenylsiloxyphenyltrimethicone | 5 | 5 | 5 | 5 |
| Diisostearyl malate | 5 | 5 | 5 | 5 |
| Octyl methoxycinnamate | 3 | 3 | 3 | 3 |
| Sorbitan sesquiisostearate | 1 | 1 | 1 | 1 |
| Long-lasting makeup effect (oily shining) | A | D | C | C |

(Production method) Cosmetic powders, oily components, an antiseptic, an ultraviolet absorber, and active agents were uniformly mixed using a pulverizer, and thus a powder cosmetic base material was produced. This was filled in a middle-sized dish and was compression molded. Thus, a foundation was obtained.

As shown in Table 1, Test Example 1 obtained by incorporating spherical PMMA particles that have been made porous in the interior and at the surfaces of the particles, had a remarkably excellent long-lasting makeup effect as compared with Test Examples 2 to 4 obtained by incorporating spherical crosslinked silicone and/or spherical porous silica.
Therefore, it is clear that the solid powder cosmetic according to the present invention exhibits a high long-lasting makeup effect by suppressing oily shining of the skin over a long time period after application.

Foundations prepared by incorporating the spherical polymethyl methacrylate particles produced in the Production Example (spherical porous PMMA, specific surface area 150 m²/g), in which the particle size was varied between 1 µm and 40 µm, were evaluated for the long-lasting makeup effect. The results are presented in the following Table 2.

**[Table 2]**

| | Test Example | | | | |
|---|---|---|---|---|---|
| | 5 | 6 | 7 | 8 | 9 |
| Silicone-treated talc | 20 | 20 | 20 | 20 | 20 |
| Silicone-treated sericite | Balance | Balance | Balance | Balance | Balance |
| Mica | 5 | 5 | 5 | 5 | 5 |
| Plate-shaped barium sulfate | 5 | 5 | 5 | 5 | 5 |
| Spherical porous PMMA average | 5 | - | - | - | - |
| particle size 1 µm | | | | | |
| Spherical porous PMMA average | - | 5 | - | - | - |
| particle size 3 µm | | | | | |
| Spherical porous PMMA average | - | - | 5 | - | - |
| particle size 10 µm | | | | | |
| Spherical porous PMMA average | - | - | - | 5 | - |
| particle size 20 µm | | | | | |
| Spherical porous PMMA average | - | - | - | - | 5 |
| particle size 40 µm | | | | | |
| Silicone-treated titanium oxide | 10 | 10 | 10 | 10 | 10 |
| Alkylsilicone-treated iron oxide | 5 | 5 | 5 | 5 | 5 |
| Titanium oxide fine particles | 5 | 5 | 5 | 5 | 5 |
| Antiseptic | 0.2 | 0.2 | 0.2 | 0.2 | 0.2 |
| Diphenylsiloxyphenyltrimethicone | 5 | 5 | 5 | 5 | 5 |
| Diisostearyl malate | 5 | 5 | 5 | 5 | 5 |
| Octyl methoxycinnamate | 3 | 3 | 3 | 3 | 3 |
| Sorbitan sesquiisostearate | 1 | 1 | 1 | 1 | 1 |
| Long-lasting makeup effect (oily shining) | E | C | B | B | D |

(Production method) Cosmetic powders, oily components, an antiseptic, an ultraviolet absorber, and active agents were uniformly mixed using a pulverizer, and thus a powder cosmetic base material was produced. This was filled in a middle-sized dish and was compression molded. Thus, a foundation was obtained.

As shown in Table 2, Test Examples 6 to 8 which used spherical porous PMMA having a particle size of 3 to 20 µm exhibited satisfactory results for both the long-lasting makeup effect and the feeling of use.
On the other hand, in Test Example 5 having a particle size of 1 µm, significant oily shining of the skin over time was recognized, and the feeling of fit to the skin as a foundation was also poor. Also, in Test Example 9 having a particle size of 40 µm, a decrease in the long-lasting makeup effect was recognized.
Therefore, it is suitable to adjust the particle size of the spherical (meth)acrylate particles to 3 to 20 µm, for the solid powder cosmetic according to the present invention.

Foundations prepared by incorporating spherical polymethyl methacrylate particles produced in the Production Example described above (spherical porous PMMA, average particle size 10 µm), in which the specific surface area was varied between 50 m²/g and 210 m²/g, were evaluated for the long-lasting makeup effect. The results are presented in the following Table 3.

**[Table 3]**

| | Test Example | | | | |
|---|---|---|---|---|---|
| | 10 | 11 | 12 | 13 | 14 |
| Silicone-treated talc | 20 | 20 | 20 | 20 | 20 |
| Silicone-treated sericite | Balance | Balance | Balance | Balance | Balance |
| Mica | 5 | 5 | 5 | 5 | 5 |
| Plate-shaped barium sulfate | 5 | 5 | 5 | 5 | 5 |
| Spherical porous PMMA specific | 5 | - | - | - | - |
| surface area 50 m²/g | | | | | |
| Spherical porous PMMA specific | - | 5 | - | - | - |
| surface area 80 m²/g | | | | | |
| Spherical porous PMMA specific | - | - | 5 | - | - |
| surface area 150 m²/g | | | | | |
| Spherical porous PMMA specific | - | - | - | 5 | - |
| surface area 180 m²/g | | | | | |
| Spherical porous PMMA specific | - | - | - | - | 5 |
| surface area 210 m²/g | | | | | |
| Silicone-treated titanium oxide | 10 | 10 | 10 | 10 | 10 |
| Alkylsilicone-treated iron oxide | 5 | 5 | 5 | 5 | 5 |
| Titanium oxide fine particles | 5 | 5 | 5 | 5 | 5 |
| Antiseptic | 0.2 | 0.2 | 0.2 | 0.2 | 0.2 |
| Diphenylsiloxyphenyltrimethicone | 5 | 5 | 5 | 5 | 5 |
| Diisostearyl malate | 5 | 5 | 5 | 5 | 5 |
| Octyl methoxycinnamate | 3 | 3 | 3 | 3 | 3 |
| Sorbitan sesquiisostearate | 1 | 1 | 1 | 1 | |
| Long-lasting makeup effect (oily shining) | E | C | B | B | D |

(Production method) Cosmetic powders, oily components, an antiseptic, an ultraviolet absorber, and active agents were uniformly mixed using a pulverizer, and thus a powder cosmetic base material was produced. This was filled in a middle-sized dish and was compression molded. Thus, a foundation was obtained.

As shown in Table 3, Test Examples 11 to 13 using spherical porous PMMA particles having a specific surface area of 80 to 180 m²/g, exhibited satisfactory results for the long-lasting makeup effect.
On the other hand, in Test Example 10 having a specific surface area of 50 m²/g and a Test Example having a specific surface area of 210 m²/g, significant oily shining of the skin over time was recognized, and the feeling of fit to the skin as a foundation was also poor.
Therefore, it is suitable to adjust the specific surface area of the spherical (meth)acrylate particles to 80 to 180 m²/g, for the solid powder cosmetic according to the present invention.

Furthermore, spherical polymethyl methacrylate particles (spherical porous PMMA particles) having an average particle size of 10 µm and a specific surface area of 150 m²/g, and having the most frequent diameter of the pores at the surfaces and in the interior of the particles varied between 100 Å and 250 Å, were produced in the Production Example described above. The respective particles were evaluated for the long-lasting makeup effect. The results are presented in the following Table 4.

**[Table 4]**

| | Test Example | | | | |
|---|---|---|---|---|---|
| | 15 | 16 | 17 | 18 | 19 |
| Silicone-treated talc | 20 | 20 | 20 | 20 | 20 |
| Silicone-treated sericite | Balance | Balance | Balance | Balance | Balance |
| Mica | 5 | 5 | 5 | 5 | 5 |
| Plate-shaped barium sulfate | 5 | 5 | 5 | 5 | 5 |
| Spherical porous PMMA most | 5 | - | - | - | - |
| frequent pore diameter 100Å | | | | | |
| Spherical porous PMMA most | - | 5 | - | - | - |
| frequent pore diameter 160Å | | | | | |
| Spherical porous PMMA most | - | - | 5 | - | - |
| frequent pore diameter 180Å | | | | | |
| Spherical porous PMMA most | - | - | - | 5 | - |
| frequent pore diameter 200Å | | | | | |
| Spherical porous PMMA most | - | - | - | - | 5 |
| frequent pore diameter 220Å | | | | | |
| Silicone-treated titanium oxide | 10 | 10 | 10 | 10 | 10 |
| Alkylsilicone-treated iron oxide | 5 | 5 | 5 | 5 | 5 |
| Titanium oxide fine particles | 5 | 5 | 5 | 5 | 5 |
| Antiseptic | 0.2 | 0.2 | 0.2 | 0.2 | 0.2 |
| Diphenylsiloxyphenyltrimethicone | 5 | 5 | 5 | 5 | 5 |
| Diisostearyl malate | 5 | 5 | 5 | 5 | 5 |
| Octyl methoxycinnamate | 3 | 3 | 3 | 3 | 3 |
| Sorbitan sesquiisostearate | 1 | 1 | 1 | 1 | 1 |
| Long-lasting makeup effect (oily | E | D | B | B | B |
| shining) | | | | | |

(Production method) Cosmetic powders, oil preparations, an antiseptic, an ultraviolet absorber, and active agents were uniformly mixed using a pulverizer, and thus a powder cosmetic base material was produced. This was filled in a middle-sized dish and was compression molded. Thus, a foundation was obtained.

As shown in Table 4, in Test Examples 17 to 19 prepared by incorporating spherical porous PMMA particles in which the most frequent pore size of the pores at the surfaces and in the interior was adjusted to 180 Å or greater, a high long-lasting makeup effect was recognized. On the contrary, Test Examples 15 and 16 which had most frequent pore sizes smaller than 180 Å, did not exhibit sufficient long-lasting makeup effects as compared with the previous Test Examples. This was speculated to be because the spherical porous PMMA particles incorporated into Test Examples 17 to 19 efficiently adsorbed large amounts of oleic acid, which is a sebum component.
Therefore, it is preferable to adjust the most frequent pore diameter of the pores at the surfaces and in the interior of the spherical (meth)acrylate particles to 180 Å or greater, for the solid powder cosmetic according to the present invention.

Foundations prepared by varying the amount of incorporation of the spherical polymethyl methacrylate particles produced in the Production Example described above (spherical porous PMMA, particle size 10 µm) between 0% and 30% by mass, were evaluated for the long-lasting makeup effect and the feeling of use. The results are presented in the following Table 5.

**[Table 5]**

| | Test Example | | | | | |
|---|---|---|---|---|---|---|
| | 20 | 21 | 22 | 23 | 24 | 25 |
| Silicone-treated talc | 20 | 20 | 20 | 20 | 20 | 20 |
| Silicone-treated sericite | Balance | Balance | Balance | Balance | Balance | Balance |
| Mica | 5 | 5 | 5 | 5 | 5 | 5 |
| Plate-shaped barium sulfate | 5 | 5 | 5 | 5 | 5 | 5 |
| Spherical porous PMMA | - | 1 | 5 | 10 | 20 | 30 |
| (particle size 10 µm, specific | | | | | | |
| surface area 100 m²/g, most | | | | | | |
| frequent pore diameter 180Å) | | | | | | |
| Silicone-treated titanium oxide | 10 | 10 | 10 | 10 | 10 | 10 |
| Alkylsilicone-treated iron oxide | 5 | 5 | 5 | 5 | 5 | 5 |
| Titanium oxide fine particles | 5 | 5 | 5 | 5 | 5 | 5 |
| Antiseptic | 0.2 | 0.2 | 0.2 | 0.2 | 0.2 | 0.2 |
| Diphenylsiloxyphenyltrimethicone | 5 | 5 | 5 | 5 | 5 | 5 |
| Diisostearyl malate | 5 | 5 | 5 | 5 | 5 | 5 |
| Octyl methoxycinnamate | 3 | 3 | 3 | 3 | 3 | 3 |
| Sorbitan sesquiisostearate | 1 | 1 | 1 | 1 | 1 | 1 |
| Long-lasting makeup effect (oily | E | C | B | B | B | B |
| shining) | | | | | | |
| Feeling of use (mealiness) | 4 | 4 | 4 | 4 | 3 | 1 |

(Production method) Cosmetic powders, oily components, an antiseptic, an ultraviolet absorber, and active agents were uniformly mixed using a pulverizer, and thus a powder cosmetic base material was produced. This was filled in a middle-sized dish and was compression molded. Thus, a foundation was obtained.

As shown in Table 5, in the foundations of Test Examples 21 to 24 obtained by incorporating spherical porous PMMA particles in an amount of 1% to 20% by mass based on the composition, oily shining was not recognized even after 3 hours, the long-lasting makeup effect was excellent, and usage properties were also maintained. On the other hand, in Test Example 20 produced without incorporating the spherical porous PMMA, oily shining of the skin over time was recognized. Furthermore, in Test Example 25 produced by 30% by mass of the spherical porous PMMA, the long-lasting makeup effect was excellent, but the feeling of use was markedly poor.
Therefore, it is suitable to adjust the amount of incorporation of the spherical (meth)acrylate particles in the solid powder cosmetic according to the present invention, to 1% to 20% by mass based on the cosmetic composition, in view of the feeling of use.

### <Use of activated zinc oxide in combination>

The following solid powder cosmetics of Test Examples 26 to 29 were produced by incorporating spherical polymethyl methacrylate particles having pores in the interior and at the surfaces (spherical porous PMMA) which are commercially available or were obtained in the Production Example described above, and activated zinc oxide, were evaluated for the long-lasting makeup effect for 30 to 10 hours after application (air temperature 32°C, humidity 65%), according to the evaluation criteria described above.
In addition, Test Example 30 produced by increasing the amount of talc (3) instead of excluding activated zinc oxide (2) from the formulation of the following Test Example 26; Test Example 31 produced by replacing the spherical porous PMMA (1) with a spherical organopolysiloxane elastomer powder (TREFIL E506C, manufactured by Dow Coming Silicone Co., Ltd.); Test Example 32 produced by increasing the amount of talc (3) instead of excluding the spherical porous PMMA (1) from the formulation; and Test Example 33 produced by replacing the spherical porous PMMA (1) of the following Test Example 29 with poreless, true spherical PMMA (8 µm), were also evaluated in the same manner. The results are presented in Table 6.

### <Test Example 26: Pressed powder>

| (Component) | (mass%) |
|---|---|
| (1) Spherical porous PMMA | 5 |
| (average particle size 8 µm, specific surface area 150 m²/g, most frequent pore diameter 180 Å: TECHPOLYMER MBP-8HP, manufactured by Sekisui Chemical Co., Ltd.) | |
| (2) Activated zinc oxide | 5 |
| (activated zinc oxide AZO-B, manufactured by Seido Chemical Industry Co., Ltd.) | |
| (3) Talc | Balance |
| (4) Titanium oxide | 7.5 |
| (5) Bengala | 0.6 |
| (6) Yellow iron oxide | 1.7 |
| (7) Black iron oxide | 006 |
| (8) Antiseptic | 0.2 |
| (9) Diisostearyl malate | 1 |
| (10) Glyceryl tri-2-ethylhexanoate | 2 |
| (11) Octyl methoxycinnamate | 4 |
| (12) Sorbitan sesquiisostearate | 1 |
| (13) Castor oil | 1 |
| (14) Antioxidant | q.s |
| (15) Fragrance | q.s. |
| (Production method) | |

Cosmetic powders, oily components, surfactants, and an antioxidant were uniformly mixed using a pulverizer, and thus a powder cosmetic base material was produced. This was filled in a middle-sized dish and was compression molded to obtain a pressed powder.

### <Test Example 27: Powdery foundation>

| (Component) | (mass%) |
|---|---|
| (1) Spherical porous PMMA | 15 |
| (average particle size 8 µm, specific surface area 150 m²/g, most frequent pore diameter 180 Å: TECHPOLYMER MBP-8HP, manufactured by Sekisui Chemical Co., Ltd.) | |
| (2) Activated zinc oxide | 5 |
| (activated zinc oxide AZO-B, manufactured by Seido Chemical Industry Co., Ltd.) | |
| (3) Talc | 15 |
| (4) Mica | 10 |
| (5) Sericite | 15 |
| (6) Titanium oxide | 10 |
| (7) Bengala | 0.6 |
| (8) Yellow iron oxide | 1.9 |
| (9) Black iron oxide | 0.15 |
| (10) Antiseptic | 0.4 |
| (11) Dimethylpolysiloxane | 1 |
| (12) Diisostearyl malate | 5 |
| (13) Trimethylpropane triisostearate | 5 |
| (14) Sorbitan sesquiisostearate | 1 |
| (15) Castor oil | q.s. |
| (16) Diisostearyl malate | q.s. |
| (17) Antioxidant | q.s. |
| (18) Fragrance | q.s. |
| (Production method) | |

Cosmetic powders, oily components, surfactants, and an antioxidant were uniformly mixed using a pulverizer, and thus a powder cosmetic base material was produced. This was filled in a middle-sized dish and was compression molded to obtain a powdery foundation.

### <Test Example 28: Dual foundation>

| (Component) | (mass%) |
|---|---|
| (1) Spherical porous PMMA | 10 |
| (porous powder having an average particle size of 8 µm, a specific surface area of 150 m²/g, and a most frequent pore diameter of 180 Å, produced in the Production Example described above) | |
| (2) Activated zinc oxide | 10 |
| (activated zinc oxide AZO-B, manufactured by Seido Chemical Industry Co., Ltd.) | |
| (3) Silicone-treated talc | 14.5 |
| (4) Silicone-treated mica | 10 |
| (5) Silicone-treated sericite | 15 |
| (6) Silicone-treated titanium dioxide | 10 |
| (7) Silicone-treated color pigment | 5 |
| (8) Antiseptic | 0.4 |
| (9) Squalane | 3 |
| (10) Solid paraffin | 1 |
| (11) Dimethylpolysiloxane | 4 |
| (12) Octyl methoxycinnamate | 1 |
| (13) Castor oil | 2 |
| (14) Antioxidant | q.s. |
| (15) Fragrance | q.s. |
| (Production method) | |

Cosmetic powders, oily components, surfactants, and an antioxidant were uniformly mixed using a pulverizer, and thus a powder cosmetic base material was produced. This was filled in a middle-sized dish and was compression molded to obtain a dual foundation.

### <Test Example 29: Foundation>

| (Component) | (mass%) |
|---|---|
| (1) Spherical porous PMMA | 5 |
| (porous powder having an average particle size of 8 µm, a specific surface area of 150 m²/g, and a most frequent pore diameter of 180 A, produced in the Production Example described above) | |
| (2) Activated zinc oxide | 5 |
| (activated zinc oxide AZO-B, manufactured by Seido Chemical Industry Co., Ltd.) | |
| (3) Silicone-treated talc | 15 |
| (4) Silicone-treated sericite | Balance |
| (5) Mica | 5 |
| (6) Plate-shaped barium sulfate | 5 |
| (7) Silicone-treated titanium oxide | 10 |
| (8) Alkylsilicone-treated iron oxide | 5 |
| (9) Titanium oxide fine particles | 5 |
| (10) Antiseptic | 0.2 |
| (11) Diphenylsiloxyphenyltrimethicone | 0.5 |
| (12) Diisostearyl malate | 5 |
| (13) Octyl methoxycinnamate | 3 |
| (14) Sorbitan sesquiisostearate | 1 |
| (Production method) | |

Cosmetic powders, oily components, an antiseptic, an ultraviolet absorber, and active agents were uniformly mixed using a pulverizer, and thus a powder cosmetic base material was produced. This was filled in a middle-sized dish and was compression molded to obtain a foundation.

**[Table 6]**

| | Test Example | | | | | | | |
|---|---|---|---|---|---|---|---|---|
| | 26 | 27 | 28 | 29 | 30 | 31 | 32 | 33 |
| After 30 minutes | A | A | A | A | A | B | D | D |
| After 2 hours | A | A | A | A | A | B | D | D |
| After 5 hours | A | A | A | A | B | D | D | D |
| After 10 hours | B | B | B | B | D | D | D | E |

As shown in Table 6, Test Examples 26 to 29 produced by incorporating activated zinc oxide and spherical polymethyl methacrylate particles having pores in the interior and at the surfaces (spherical porous PMMA), exhibited an excellent long-lasting makeup effect without causing oily shining of the skin over a long time period.
On the other hand, Test Example 30 produced by incorporating spherical polymethyl methacrylate particles having pores in the interior and at the surfaces, but not incorporating activated zinc oxide, exhibited an excellent long-lasting makeup effect until 5 hours after application. However, in an evaluation after 10 hours, oily shining of the skin was recognized. Furthermore, Test Example 31 produced by incorporating another porous spherical resin powder together with activated zinc oxide was slightly excellent in the short-term long-lasting makeup effect, but 5 hours after application, makeup smudging was recognized. Test Example 32 produced by incorporating activated zinc oxide but not incorporating spherical polymethyl methacrylate particles; and Test Example 33 produced using poreless spherical polymethyl methacrylate particles, exhibited almost no long-lasting makeup effect.
Therefore, it is suitable to use spherical (meth)acrylate particles and activated zinc oxide in combination for the solid powder cosmetic according to the present invention, from the viewpoint of a long-lasting makeup effect for a long time period.

Hereinafter, another Test Example (face powder) of the solid powder cosmetic according to the present invention will be shown in the following Table 7. The production of the face powder was carried out by a routine method, and the test and evaluation methods were the same as described above.

**[Table 7]**

| | Test Example | | |
|---|---|---|---|
| | 34 | 35 | 36 |
| Activated zinc oxide | 5 | 5 | - |
| Spherical porous PMMA | 5 | - | - |
| (porous powder having an average particle size of 10 µm and a specific surface area of 150 m²/g as in the Production Example) | | | |
| Glycerol-modified silicone resin-coated sericite | 0.1 | 0.1 | 0.1 |
| Yellow iron oxide-coated mica titanium | 0.1 | 0.1 | 0.1 |
| Talc | Balance | Balance | Balance |
| Mica | 20 | 20 | 20 |
| Boron nitride | 0.1 | 0.1 | 0.1 |
| Zinc myristate | 3 | 3 | 3 |
| Aluminum stearate | 0.1 | 0.1 | 0.1 |
| Crosslinked type silicone powder | 2 | 2 | 2 |
| Silicic anhydride | 6 | 6 | 6 |
| Bengala-coated mica titanium | 0.1 | 0.1 | 0.1 |
| Ascorbyl dipalmitate | 0.01 | 0.01 | 0.01 |
| Tocopherol acetate | 0.02 | 0.02 | 0.02 |
| Paraoxybenzoic acid ester | q.s. | q.s. | q.s. |
| Bengala-coated mica titanium | 0.1 | 0.1 | 0.1 |
| Yellow iron oxide | 0.1 | 0.1 | 0.1 |
| Coloring | q.s. | q.s. | q.s. |
| Fragrance | q.s. | q.s. | q.s. |
| Long-lasting makeup effect (oily shining) | A | D | E |
| Feeling of use (mealiness) | 4 | 4 | 2 |

(Production method) Cosmetic powders, oily components, an antiseptic, an ultraviolet absorber, and active agents were uniformly mixed using a pulverizer, and thus a powder cosmetic base material was produced. This was filled in a middle-sized dish and was compression molded to obtain a foundation.

As shown in Table 7 above, Test Example 34 produced by incorporating activated zinc oxide and spherical porous PMMA having pores in the interior and at the surfaces, exhibited excellent results for both the long-lasting makeup effect and the feeling of use, as compared with test Example 35 produced by incorporating activated zinc oxide only, and Test Example 36 produced without incorporating both the activated zinc oxide and the spherical porous PMMA.

### <Use of fluorine compound-treated powder in combination>

Subsequently, foundations produced by incorporating a powder component that has been surface-treated with a fluorine compound, were evaluated for water repellency and oil repellency, and the long-lasting makeup effect (dulling of color). The evaluation criteria were as follows. The results are presented in the following Table 8.

### Water repellency and oil repellency

An observation was made on water repellency by applying a foundation on a surface coated with a water-insoluble composite film, and dropping 20 mg of water droplets thereon. Furthermore, an observation was made on oil repellency by applying the foundation on a surface coated with an oil-insoluble composite, and dropping 20 mg of oleic acid or squalene. The results of the observations were judged according to the following evaluation criteria.
A: The foundation was highly repellent to water and oil.
B: The foundation was slightly wetted by any one of water and oil.
C: The foundation was wetted by any one of water and oil.
D: The foundation was wetted by both water and oil.

### Long-lasting makeup effect (dulling of color)

A panel of twenty female testers applied the foundation of each Test Example on a half of the face, and performed a comparative evaluation on the dulling of color after a lapse of 3 hours.

| | |
|---|---|
| 17 or more people answered good | A |
| 12 to 16 people | B |
| 9 to 11 people | C |
| 5 to 8 people | D |
| 4 or fewer people | E |

**[Table 8]**

| | Test Example | | |
|---|---|---|---|
| | 37 | 38 | 39 |
| 1H,1H,2H,2H-perfluorooctyltriethoxysilane | 20 | - | - |
| (5%)-treated talc | | | |
| 1H,1H,2H,2H-perfluorooctyltriethoxysilane | Balance | - | - |
| (5%)-treated sericite | | | |
| 1H, 1H,2H,2H-perfluorooctyltriethoxysilane | 5 | - | - |
| (5%)-treated mica | | | |
| 1H, 1H,2H,2H-perfluorooctyltriethoxysilane | - | 20 | - |
| (5%)/acrylic-silicone copolymer (formula (V)) | | | |
| (2%)-treated talc | | | |
| 1H,1H,2H,2H-perfluorooctyltriethoxysilane | - | Balance | - |
| (5%)/acrylic-silicone copolymer (formula (V)) | | | |
| (2%)-treated sericite | | | |
| 1H,1H,2H,2H-perfluorooctyltriethoxysilane | - | 5 | - |
| (5%)/acrylic-silicone copolymer (formula (V)) | | | |
| (2%)-treated mica | | | |
| Octyltriethoxysilane-treated talc | - | - | 20 |
| Octyltriethoxysilane-treated sericite | - | - | Balance |
| Octyltriethoxysilane-treated mica | - | - | 5 |
| Plate-shaped barium sulfate | 5 | 5 | 5 |
| Spherical porous PMMA | 10 | 10 | 10 |
| (average particle size 10 µm, specific surface | | | |
| area 150 m2/g) | | | |
| Silicone-treated titanium oxide | 10 | 10 | 10 |
| Alkylsilicone-treated iron oxide | 5 | 5 | 5 |
| Ttianium oxide fine particles | 5 | 5 | 5 |
| Antiseptic | 0.2 | 0.2 | 0.2 |
| Diphenylsiloxyphenyltrimethicone | 5 | 5 | 5 |
| Diisostearyl malate | 5 | 5 | 5 |
| Octyl methoxycinnamate | 3 | 3 | 3 |
| Sorbitan sesquiisostearate | 1 | 1 | 1 |
| Water repellency/oil repellency | A | A | C |
| Long-lasting makeup effect (dulling of color) | A | A | C |

(Production method) Cosmetic powders, oily components, an antiseptic, an ultraviolet absorber, and active agents were uniformly mixed using a pulverizer, and thus a powder cosmetic base material was produced. This was filled in a middle-sized dish and was compression molded to obtain a foundation.

As shown in Table 8, the foundations of Test Examples 37 and 38 produced by incorporating a powder surface-treated with a fluorine compound in an amount of about 50% based on the composition, exhibited excellent water repellency and oil repellency, and also, dulling of color over time almost did not occur. On the contrary, in Test Example 39 produced by incorporating a powder surface-treated with octyltriethoxysilane in the same amount, the foundation did not have sufficient water repellency and oil repellency, and was not considered to be satisfactory in terms of the dulling of color over time.
As seen from these results, it is suitable to incorporate a fluorine compound-treated powder together with the porous spherical (meth)acrylate into the solid powder cosmetic according to the present invention.

In the following, other Test Examples of the solid powder cosmeticl according to the present invention will be shown below.

### <Test Example 40: Foundation>

| (Component) | (mass%) |
|---|---|
| (1) Spherical porous PMMA | 5 |
| (average particle size 8 µm, specific surface area 150 m²/g, most frequent pore diameter 180Å: TECHPOLYMER MBP-8HP, manufactured by Sekisui Chemical Co., Ltd.) | |
| (2) Activated zinc oxide | 5 |
| (activated zinc oxide AZO-B, manufactured by Seido Chemical Industry Co., Ltd.) | |
| (3) 1H, 1H,2H,2H-perfluorooctyltriethoxysilane (5%)-treated talc | |
| | Balance |
| (4) 1H, 1H,2H,2H-perfluorooctyltriethoxysilane (5%)-treated mica | |
| | 10 |
| (5) 1H,1H,2H,2H-perfluorooctyltriethoxysilane (5%)-treated sericite | |
| | 15 |
| (6) Silicone-treated titanium dioxide | 10 |
| (7) Silicone-treated color pigment | 5 |
| (8) Antiseptic | 0.4 |
| (9) Methylphenylpolysiloxane | 2 |
| (10) Squalane | 1 |
| (11) Dimethylpolysiloxane | 4 |
| (12) Octyl methoxycinnamate | 2 |
| (13) Surfactant | 1 |
| (14) Antioxidant | q.s. |
| (15) Fragrance | q.s. |
| (Production method) | |

Cosmetic powders, oily components, an antiseptic, an ultraviolet absorber, and active agents were uniformly mixed using a pulverizer, and thus a powder cosmetic base material was produced. This was filled in a middle-sized dish and was compression molded to obtain a pressed powder.

The solid powder cosmetic obtained in Test Example 40 had an excellent long-lasting makeup effect (oily shining, and dulling of color) and a satisfactory feeling of use (feeling of particulateness or the like).

### <Production using facing rotor type mixing apparatus>

Subsequently, a foundation produced by mixing powder components and oily components using a facing rotor type mixing apparatus, was evaluated for the feelings of use (feeling of particulate fineness, moist feeling, and smoothness) and impact resistance. The evaluation criteria are as follows. The results are presented in the following Tables 9 to 11.

### Impact resistance

The foundation of each Test Example was press molded in a resin and was mounted in a compact container for cosmetic use. This was used as a sample. The sample was dropped, while in a state of being laid horizontally, from a height of 30 cm on an iron plate having a thickness of 20 mm, and the number of droppings until breakage was used as an evaluation for impact resistance.

### Feelings of use (feeling of particulate fineness, moist feeling, smoothness)

A panel of twenty female testers applied the foundation of each Test Example on a half of the face, and performed a comparative evaluation on the feelings of particulate fineness upon application, moist feeling, and smoothness.

| | |
|---|---|
| 17 or more people answered good | A |
| 12 to 16 people | B |
| 9 to 11 I people | C |
| 5 to 8 people | D |
| 4 or fewer people | E |

**[Table 9]**

| | Test Example | |
|---|---|---|
| | 41 | 42 |
| Sericite | 5 | 5 |
| Synthetic mica | 15 | 15 |
| Talc | Balance | Balance |
| Titanium oxide | 11 | 11 |
| Red interference pearl pigment | 2 | 2 |
| Activated zinc oxide | 2 | 2 |
| Bengala | 0.8 | 0.8 |
| Yellow iron oxide | 2 | 2 |
| Black iron oxide | 0.1 | 0.1 |
| Spherical silicone-based elastomeric powder | 6 | 6 |
| Spherical nylon powder | 4 | 4 |
| Spherical porous PMMA | 6 | 6 |
| (average particle size 10 µm, specific surface area 150 m²/g) | | |
| Dimethylpolysiloxane (5 mPa·s) | 3 | 3 |
| Dimethylpolysiloxane (5000 mPa·s) | 2 | 2 |
| Squalane | 3 | 3 |
| Petrolatum | 2 | 2 |
| Sorbitan sesquiisostearate | 1 | |
| Paraben | q.s. | q.s. |
| Antioxidant | q.s. | q.s. |
| Fragrance | q.s. | q.s.. |
| Production method | Preliminarily mixed in Henschel mixer | Preliminarily mixed in Henschel mixer |
| | ↓ | ↓ |
| | Mixed two times with facing rotor type mixing apparatus | Mixed two times with pulverizier ↓ |
| | ↓ | |
| | Dry press molded in middle-sized resin dish | Dry press molded in middle-sized resin dish |
| Impact resistance | 14 times | 3 times |
| Feeling of use (Fine particulate feeling) | B | C |
| (Moist feeling) | A | C |
| (Smoothness) | A | B |

### (Production method)

Test Example 41: The oily components were added to the powder components of the formulation, and the components were mixed for a certain time in a Henschel mixer (manufactured by Mitsui Miike Machinery Co., Ltd.). Subsequently, the mixture was mixed two times using the facing rotor type mixing apparatus of Fig. 1 (cyclone mill: manufactured by Flo-tec, Ltd.; used by rotating the first rotor and the second rotor in the opposite direction to each other). The mixture was dry press molded in a middle-sized resin dish.
Test Example 42: The oily components were added to the powder components of the formulation, and the components were mixed in a Henschel mixer. Subsequently, the mixture was mixed two times with a pulverizer (manufactured by Hosokawa Micron Corp.), which is a hammer type pulverizing machine, and was dry press molded in a middle-sized resin dish.

**[Table 10]**

| | Test Example | |
|---|---|---|
| | 43 | 44 |
| Silicone-treated sericite | 12 | 12 |
| Silicone-treated mica | Balance | Balance |
| Silicone-treated titanium oxide | 23 | 23 |
| Aluminum stearate-treated titanium oxide fine particles | 12 | 12 |
| Silicone-treated Bengala | 4 | 4 |
| Silicone-treated yellow iron oxide | 1.2 | 1.2 |
| Silicone-treated black iron oxide | 2.5 | 2.5 |
| Polyurethane powder | 0.9 | 0.9 |
| Spherical porous PMMA | 5 | 5 |
| (particle size 10 µm, specific surface area 150 m²/g) | | |
| Fine zinc oxide | 2 | 2 |
| Paraben | q.s. | q.s. |
| Dimethylpolysiloxane (5 mPa·s) | 3 | 3 |
| Hydrogenated polyisobutene (5000 mPa·s) | 2 | 2 |
| Methylphenylpolysiloxane | 3 | 3 |
| Octyl methoxycinnamate | 3 | 3 |
| Polyether-modified silicone | 1 | 1 |
| Antioxidant | q.s. | q.s. |
| Fragrance | q.s. | q.s. |
| Production method | Preliminarily mixed in Henschel mixer | Preliminarily mixed in Henschel mixer |
| | ↓ | ↓ |
| | Mixed two times with facing rotor type mixing apparatus | Mixed two times with pulverizier ↓ |
| | ↓ | |
| | Dry press molded in middle-sized resin dish | Dry press molded in middle-sized resin dish |
| Impact resistance | 10 times | 4 times |
| Feeling of use (Fine particulate feeling) | A | D |
| (Moist feeling) | B | C |
| (Smoothness) | A | C |

### (Production method)

Test Example 43: The oily components were added to the powder components of the formulation, and the components were mixed for a certain time in a Henschel mixer (manufactured by Mitsui Miike Machinery Co., Ltd.). Subsequently, the mixture was mixed two times using the facing rotor type mixing apparatus of Fig. 1 (cyclone mill: manufactured by Flo-tec, Ltd.; used by rotating the first rotor and the second rotor in the opposite direction to each other). The mixture was dry press molded in a middle-sized resin dish.
Test Example 44: The oily components were added to the powder components of the formulation, and the components were mixed in a Henschel mixer. Subsequently, the mixture was mixed two times with a pulverizer (manufactured by Hosokawa Micron Corp.), which is a hammer type pulverizing machine, and was dry press molded in a middle-sized resin dish.

**[Table 11]**

| | Test Example | |
|---|---|---|
| | 45 | 46 |
| 1H, 1H,2H,2H-perfluorooctyltriethoxysilane | 5 | 5 |
| (5%)/acrylic-silicone copolymer (formula | | |
| (V)) (2%)-treated sericite | | |
| 1H, H,2H,2H-perfluorooctyltriethoxysilane | Balance | Balance |
| (5%)/acrylic-silicone copolymer (formula | | |
| (V)) (2%)-treated mica | | |
| 1H, H,2H,2H-perfluorooctyltriethoxysilane | 28 | 28 |
| (5%)/acrylic-silicone copolymer (formula | | |
| (V)) (2%)-treated talc | | |
| 1H,1H,2H,2H-perfluorooctyltnethoxysilane | 12 | 12 |
| (5%)/acrylic-silicone copolymer (formula | | |
| (V)) (2%)-treated titanium oxide fine | | |
| particles | | |
| Aluminum stearate-treated titanium oxide | 4 | 4 |
| fine particles | | |
| 1H,1H,2H,2H-perfluorooctyltriethoxysilane | 1.2 | 1.2 |
| (5%)/acrylic-silicone copolymer (formula | | |
| (V)) (2%)-treated Bengala | | |
| 1H, 1H,2H,2H-perfluorooctyltriethoxysilane | 2.5 | 2.5 |
| (5%)/acrylic-silicone copolymer (formula | | |
| (V)) (2%)-treated yellow iron oxide | | |
| 1H, 1H,2H,2H-perfluorooctyltriethoxysilane | 0.9 | 0.9 |
| (5%)/acrylic-silicone copolymer (formula | | |
| (V)) (2%)-treated black iron oxide | | |
| Silicone-based elastomeric powder | 6 | 6 |
| Spherical polyethylene powder | 3 | 3 |
| Spherical porous PMMA | 5 | 5 |
| (average particle size 10 µm, specific | | |
| surface area 150 m²/g) | | |
| Fine zinc oxide | 7 | 7 |
| Fine silica | 3 | 3 |
| Paraben | q.s. | q.s. |
| Dimethylpolysiloxane (5 mPa·s) | 3 | 3 |
| Dimethylpolysiloxane (5000 mPa·s) | 3 | 3 |
| Methylphenylpolysiloxane | 2 | 2 |
| Octyl methoxycinnamate | 3 | 3 |
| Polyether-modified silicone | 1 | 1 |
| Antioxidant | q.s. | q.s. |
| Fragrance | q.s. | q.s. |
| Production method | Preliminarily mixed in | Preliminarily mixed in |
| | Henschel mixer | Henschel mixer |
| | ↓ | ↓ |
| | Mixed two times with facing rotor type mixing | Mixed two times with pulverizier |
| | apparatus | ↓ |
| | ↓ | |
| | Dry press molded in middle-sized resin dish | Dry press molded in middle-sized resin dish |
| Impact resistance | 10 times | 2 times |
| Feeling of use (Fine particulate feeling) | A | B |
| (Moist feeling) | B | C |
| (Smoothness) | A | B |

### (Production method)

Test Example 45: The oily components were added to the powder components of the formulation, and the components were mixed for a certain time in a Henschel mixer (manufactured by Mitsui Miike Machinery Co., Ltd.). Subsequently, the mixture was mixed two times using the facing rotor type mixing apparatus of Fig. 1 (cyclone mill: manufactured by Flo-tec, Ltd.; used by rotating the first rotor and the second rotor in the opposite direction to each other). The mixture was dry press molded in a middle-sized resin dish.
Test Example 46: The oily components were added to the powder components of the formulation, and the components were mixed in a Henschel mixer. Subsequently, the mixture was mixed two times with a pulverizer (manufactured by Hosokawa Micron Corp.), which is a hammer type pulverizing machine, and was dry press molded in a middle-sized resin dish.

As shown in Tables 9 to 11, the foundations of Test Examples 41, 43 and 45 produced using a facing rotor type mixing apparatus had remarkably improved impact resistance as compared with the Test Examples 42, 44 and 46 produced using a pulverizer (hammer type pulverizing machine). Furthermore, it was found that the foundations of Test Examples 41, 43 and 45 were also excellent in the feelings of use such as the feeling of particulate fineness, the moist feeling and smoothness.

The same evaluation was performed with foundations produced by using a facing rotor type mixing apparatus in the same manner as in the above-described test, and varying the amount of incorporation of the spherical polymethyl methacrylate particles (spherical porous PMMA, particle size 10 µm) between 1% to 20% by mass. The results are presented in the following Table 12.

**[Table 12]**

| | Test Example | | | | |
|---|---|---|---|---|---|
| | 47 | 48 | 49 | 50 | 51 |
| Silicone-treated sericite | 12 | 12 | 12 | 12 | 12 |
| Silicone-treated mica | Balance | Balance | Balance | Balance | Balance |
| Silicone-treated titanium oxide | 23 | 23 | 23 | 23 | 23 |
| Aluminum stearate-treated titanium oxide fine particles | 12 | 12 | 12 | 12 | 12 |
| Silicone-treated Bengala | 4 | 4 | 4 | 4 | 4 |
| Silicone-treated yellow iron oxide | 1.2 | 1.2 | 1.2 | 1.2 | 1.2 |
| Silicone-treated black iron oxide | 2.5 | 2.5 | 2.5 | 2.5 | 2.5 |
| Polyurethane powder | 0.9 | 0.9 | 0.9 | 0.9 | 0.9 |
| Spherical porous PMMA | 1 | 6 | 10 | 15 | 20 |
| (average particle size 10 µm, specific surface area 150 m²/g) | | | | | |
| Fine zinc oxide | 2 | 2 | 2 | 2 | 2 |
| Paraben | q.s. | q.s. | q.s. | q.s. | q.s. |
| Dimethylpolysiloxane (5 mPa·s) | 3 | 3 | 3 | 3 | 3 |
| Hydrogenated polyisobutene (5000 mPa·s) | 2 | 2 | 2 | 2 | 2 |
| Methylphenylpolysiloxane | 3 | 3 | 3 | 3 | 3 |
| Octyl methoxycinnamate | 3 | 3 | 3 | 3 | 3 |
| Polyether-modified silicone | 1 | 1 | 1 | 1 | 1 |
| Antioxidant | q.s. | q.s. | q.s. | q.s. | q.s. |
| Fragrance | q.s. | q.s. | q.s. | q.s. | q.s. |
| Production method | Preliminarily mixed in Henschel mixer | | | | |
| | ↓ | | | | |
| | Mixed two times with facing rotor type mixing apparatus | | | | |
| | ↓ | | | | |
| | Dry press molded in middle-sized resin dish | | | | |
| Impact resistance | 20 times | 14 times | 10 times | 7 times | 4 times |
| Feeling of use (Fine particulate feeling) | A | A | A | A | A |
| (Moist feeling) | B | B | B | C | D |
| (Smoothness) | A | A | A | B | B |

### (Production method)

Test Examples 47 to 51: The oily components were added to the powder components of the formulation, and the components were mixed for a certain time in a Henschel mixer (manufactured by Mitsui Miike Machinery Co., Ltd.). Subsequently, the mixture was mixed two times using the facing rotor type mixing apparatus of Fig. 1 (cyclone mill: manufactured by Flo-tec, Ltd.; used by rotating the first rotor and the second rotor in the opposite direction to each other). The mixture was dry press molded in a middle-sized resin dish.

As shown in Table 12 above, the foundations of Test Examples 47 to 49 produced by incorporating 1 to 10% by mass of the spherical polymethyl methacrylate particles were excellent in the impact resistance and various feelings of use (feeling of particulate fineness, moist feeling, and smoothness). On the other hand, in the Test Examples 50 and 51 produced by incorporating 15% by mass or more of the spherical polymethyl methacrylate particles, the moist feeling could not be obtained, and the foundations tended to be deteriorated in impact resistance as well.
As shown from these results, particularly in the case of producing the solid powder cosmetic of the present invention using a facing rotor type mixing apparatus, it is suitable to adjust the amount of incorporation of the spherical polymethyl methacrylate in an amount of 1 % to 10% by mass.

In the following, other Test Examples of the solid powder cosmetic according to the present invention will be described below.

### <Test Example 52: Foundation>

| (Component) | (mass%) |
|---|---|
| Sericite | 10 |
| Synthetic mica | Balance |
| Zinc decyltrisiloxane carboxylate-coated talc | 5 |
| Spherical porous PMMA powder | 5 |
| (particle size 10 µm, specific surface area 150 m²/g) | |
| Spherical silicone powder | 3 |
| (TREFIL E-506S: manufactured by Dow Coming Toray Co., Ltd.) | |
| Spherical silicone powder | 5 |
| (TOSPEARL 2000B: manufactured by Toshiba Silicones Co., Ltd.) | |
| Spherical silicone powder | 5 |
| (SILICONE POWDER KSP300: manufactured by Shin-Etsu Chemical Co., Ltd.) | |
| Spherical porous silica | 2 |
| (SUNSPHERE L-51: manufactured by Asahi Glass Co., Ltd.) | |
| Silicone-treated titanium oxide | 15 |
| Red interference pearl pigment | 2 |
| Zinc oxide | 2 |
| Silicone-treated Bengala | 0.8 |
| Silicone-treated yellow iron oxide | 2 |
| Silicone-treated black iron oxide | 0.1 |
| Spherical nylon powder | 4 |
| 1H,1H,2H,2H-perfluorooctyltriethoxysilane (5%)/acrylic-silicone copolymer | |
| (formula (V)) (2%)-treated talc | 20 |
| 1H,1H,2H,2H-perfluorooctyltriethoxysilane (5%)/acrylic-silicone copolymer | |
| (formula (V)) (2%)-treated barium sulfate | 10 |
| Dimethylpolysiloxane (5 mPa·s) | 3 |
| Dimethylpolysiloxane (5000 mPa·s) | 2 |
| Squalane | 3 |
| Petrolatum | 1 |
| Di(phytosteryl-behenyl) dimer dilinoleate | 2 |
| Sorbitan sesquiisostearate | 0.2 |
| Chlorphenesin | q.s. |
| Antioxidant | q.s. |
| Fragrance | q.s. |
| (Production method) | |

The oily components were added to the powder components of the formulation, and the components were mixed for a certain time in a Henschel mixer (manufactured by Mitsui Miike Machinery Co., Ltd.). Subsequently, the mixture was mixed two times using the facing rotor type mixing apparatus of Fig. 1 (cyclone mill: manufactured by Flo-tec, Ltd.; used by rotating the first rotor and the second rotor in the opposite direction to each other). The mixture was dry press molded in a middle-sized resin dish.

### <Test Example 53: White powder (pressed powder)>

| (Component) | (mass%) |
|---|---|
| Zinc decyltrisiloxanecarboxylate-coated talc | 10 |
| Synthetic mica | Balance |
| Silylated silica | 5 |
| Zinc oxide | 5 |
| Red interference pearl pigment | 3 |
| Titanium oxide fine particles | 3 |
| Spherical porous PMMA powder | 5 |
| (particle size 10 µm, specific surface area 150 m²/g) | |
| Spherical silicone powder | 3 |
| (TREFIL E506S: Dow Coming Toray Co., Ltd.) | |
| Spherical silicone powder | 5 |
| (TOSPEARL 2000B: manufactured by Toshiba Silicones Co., Ltd.) | |
| Spherical silicone powder | 5 |
| (SILICONE POWDER KSP300: manufactured by Shin-Etsu Chemical Co., Ltd.) | |
| Spherical porous silica | 2 |
| (SUNSPHERE L-51: manufactured by Asahi Glass Co., Ltd.) | |
| 1H,1H,2H,2H-perfluorooctyltriethoxysilane (5%)-treated talc | 40 |
| Hydrogenated polyisobutene (20000 mPa·s) | 0.5 |
| Diisostearyl malate (2000 mPa·s) | 1 |
| Squalane | 1 |
| Ester oil | 1 |
| Paraben | q. s. |
| Antioxidant | q.s. |
| Fragrance | q.s. |

(Production method) The oily components were added to the powder components of the formulation, and the components were mixed for a certain time in a Henschel mixer (manufactured by Mitsui Miike Machinery Co., Ltd.). Subsequently, the mixture was mixed two times using the facing rotor type mixing apparatus of Fig. 1 (cyclone mill: manufactured by Flo-tec, Ltd.; used by rotating the first rotor and the second rotor in the opposite direction to each other). The mixture was dry press molded in a middle-sized resin dish.

The solid powder cosmetics obtained in Test Examples 52 and 53 as described above all had satisfactory long-lasting makeup effects (oily shining) and satisfactory feelings of use (feeling of mealiness, dulling of color over time, feeling of particulate fineness, moist feeling, smoothness, and the like), and were very excellent in terms of impact resistance as well. invention exhibits a high long-lasting makeup effect by suppressing oily shining of the skin over a long time period after application.

Foundations prepared by incorporating the spherical polymethyl methacrylate particles produced in the Production Example (spherical porous PMMA, specific surface area 150 m²/g), in which the particle size was varied between 1 µm and 40 µm, were evaluated for the long-lasting makeup effect. The results are presented in the following Table 2.

**[Table 2]**

| | Test Example | | | | |
|---|---|---|---|---|---|
| | 5 | 6 | 7 | 8 | 9 |
| Silicone-treated talc | 20 | 20 | 20 | 20 | 20 |
| Silicone-treated sericite | Balance | Balance | Balance | Balance | Balance |
| Mica | 5 | 5 | 5 | 5 | 5 |
| Plate-shaped barium sulfate | 5 | 5 | 5 | 5 | 5 |
| Spherical porous PMMA average | 5 | - | - | - | - |
| particle size 1 µm | | | | | |
| Spherical porous PMMA average | - | 5 | - | - | - |
| particle size 3 µm | | | | | |
| Spherical porous PMMA average | - | - | 5 | - | - |
| particle size 10 µm | | | | | |
| Spherical porous PMMA average | - | - | - | 5 | - |
| particle size 20 µm | | | | | |
| Spherical porous PMMA average | - | - | - | - | 5 |
| particle size 40 µm | | | | | |
| Silicone-treated titanium oxide | 10 | 10 | 10 | 10 | 10 |
| Alkylsilicone-treated iron oxide | 5 | 5 | 5 | 5 | 5 |
| Titanium oxide fine particles | 5 | 5 | 5 | 5 | 5 |
| Antiseptic | 0.2 | 0.2 | 0.2 | 0.2 | 0.2 |
| Diphenylsiloxyphenyltrimethicone | 5 | 5 | 5 | 5 | 5 |
| Diisostearyl malate | 5 | 5 | 5 | 5 | 5 |
| Octyl methoxycinnamate | 3 | 3 | 3 | 3 | 3 |
| Sorbitan sesquiisostearate | 1 | 1 | 1 | 1 | 1 |
| Long-lasting makeup effect (oily shining) | E | C | B | B | D |

(Production method) Cosmetic powders, oily components, an antiseptic, an ultraviolet absorber, and active agents were uniformly mixed using a pulverizer, and thus a powder cosmetic base material was produced. This was filled in a middle-sized dish and

**[Table 3]**

| | Test Example | | | | |
|---|---|---|---|---|---|
| | 10 | 11 | 12 | 13 | 14 |
| Silicone-treated talc | 20 | 20 | 20 | 20 | 20 |
| Silicone-treated sericite | Balance | Balance | Balance | Balance | Balance |
| Mica | 5 | 5 | 5 | 5 | 5 |
| Plate-shaped barium sulfate | 5 | 5 | 5 | 5 | 5 |
| Spherical porous PMMA specific | 5 | - | - | - | - |
| surface area 50 m²/g | | | | | |
| Spherical porous PMMA specific | - | 5 | - | - | - |
| surface area 80 m²/g | | | | | |
| Spherical porous PMMA specific | - | - | 5 | - | - |
| surface area 150 m²/g | | | | | |
| Spherical porous PMMA specific | - | - | - | 5 | - |
| surface area 180 m²/g | | | | | |
| Spherical porous PMMA specific | - | - | - | - | 5 |
| surface area 210 m²/g | | | | | |
| Silicone-treated titanium oxide | 10 | 10 | 10 | 10 | 10 |
| Alkylsilicone-treated iron oxide | 5 | 5 | 5 | 5 | 5 |
| Titanium oxide fine particles | 5 | 5 | 5 | 5 | 5 |
| Antiseptic | 0.2 | 0.2 | 0.2 | 0.2 | 0.2 |
| Diphenylsiloxyphenyltrimethicone | 5 | 5 | 5 | 5 | 5 |
| Diisostearyl malate | 5 | 5 | 5 | 5 | 5 |
| Octyl methoxycinnamate | 3 | 3 | 3 | 3 | 3 |
| Sorbitan sesquiisostearate | 1 | 1 | 1 | 1 | 1 |
| Long-lasting makeup effect (oily shining) | E | C | B | B | D |

(Production method) Cosmetic powders, oily components, an antiseptic, an ultraviolet absorber, and active agents were uniformly mixed using a pulverizer, and thus a powder cosmetic base material was produced. This was filled in a middle-sized dish and was compression molded. Thus, a foundation was obtained.

As shown in Table 3, Test Examples 1 1 to 13 using spherical porous PMMA particles having a specific surface area of 80 to 180 m²/g, exhibited satisfactory results for the long-lasting makeup effect.
On the other hand, in Test Example 10 having a specific surface area of 50 m²/g and a Test Example having a specific surface area of 210 m²/g, significant oily shining of the

**[Table 4]**

| | Test Example | | | | |
|---|---|---|---|---|---|
| | 15 | 16 | 17 | 18 | 19 |
| Silicone-treated talc | 20 | 20 | 20 | 20 | 20 |
| Silicone-treated sericite | Balance | Balance | Balance | Balance | Balance |
| Mica | 5 | 5 | 5 | 5 | 5 |
| Plate-shaped barium sulfate | 5 | 5 | 5 | 5 | 5 |
| Spherical porous PMMA most | 5 | - | - | - | - |
| frequent pore diameter 100Å | | | | | |
| Spherical porous PMMA most | - | 5 | - | - | - |
| frequent pore diameter 160Å | | | | | |
| Spherical porous PMMA most | - | - | 5 | - | - |
| frequent pore diameter 180Å | | | | | |
| Spherical porous PMMA most | - | - | - | 5 | - |
| frequent pore diameter 200Å | | | | | |
| Spherical porous PMMA most | - | - | - | - | 5 |
| frequent pore diameter 220Å | | | | | |
| Silicone-treated titanium oxide | 10 | 10 | 10 | 10 | 10 |
| Alkylsilicone-treated iron oxide | 5 | 5 | 5 | 5 | 5 |
| Titanium oxide fine particles | 5 | 5 | 5 | 5 | 5 |
| Antiseptic | 0.2 | 0.2 | 0.2 | 0.2 | 0.2 |
| Diphenylsiloxyphenyltrimethicone | 5 | 5 | 5 | 5 | 5 |
| Diisostearyl malate | 5 | 5 | 5 | 5 | 5 |
| Octyl methoxycinnamate | 3 | 3 | 3 | 3 | 3 |
| Sorbitan sesquiisostearate | 1 | 1 | 1 | 1 | 1 |
| Long-lasting makeup effect (oily shining) | E | D | B | B | B |

(Production method) Cosmetic powders, oil preparations, an antiseptic, an ultraviolet absorber, and active agents were uniformly mixed using a pulverizer, and thus a powder cosmetic base material was produced. This was filled in a middle-sized dish and was compression molded. Thus, a foundation was obtained.

As shown in Table 4, in Test Examples 17 to 19 prepared by incorporating spherical porous PMMA particles in which the most frequent pore size of the pores at the surfaces and in the interior was adjusted to 180 Å or greater, a high long-lasting makeup effect was recognized. On the contrary, Test Examples 15 and 16 which had most frequent pore sizes smaller than 180 Å, did not exhibit sufficient long-lasting makeup effects as

**[Table 5]**

| | Test Example | | | | | |
|---|---|---|---|---|---|---|
| | 20 | 21 | 22 | 23 | 24 | 25 |
| Silicone-treated talc | 20 | 20 | 20 | 20 | 20 | 20 |
| Silicone-treated sericite | Balance | Balance | Balance | Balance | Balance | Balance |
| Mica | 5 | 5 | 5 | 5 | 5 | 5 |
| Plate-shaped barium sulfate | 5 | 5 | 5 | 5 | 5 | 5 |
| Spherical porous PMMA | - | 1 | 5 | 10 | 20 | 30 |
| (particle size 10 µm, specific | | | | | | |
| surface area 100 m²/g, most | | | | | | |
| frequent pore diameter 180Å) | | | | | | |
| Silicone-treated titanium oxide | 10 | 10 | 10 | 10 | 10 | 10 |
| Alkylsilicone-treated iron oxide | 5 | 5 | 5 | 5 | 5 | 5 |
| Titanium oxide fine particles | 5 | 5 | 5 | 5 | 5 | 5 |
| Antiseptic | 0.2 | 0.2 | 0.2 | 0.2 | 0.2 | 0.2 |
| Diphenylsiloxyphenyltrimethicone | 5 | 5 | 5 | 5 | 5 | 5 |
| Diisostearyl malate | 5 | 5 | 5 | 5 | 5 | 5 |
| Octyl methoxycinnamate | 3 | 3 | 3 | 3 | 3 | 3 |
| Sorbitan sesquiisostearate | 1 | 1 | 1 | 1 | 1 | 1 |
| Long-lasting makeup effect (oily shining) | E | C | B | B | B | B |
| Feeling of use (mealiness) | 4 | 4 | 4 | 4 | 3 | 1 |

(Production method) Cosmetic powders, oily components, an antiseptic, an ultraviolet absorber, and active agents were uniformly mixed using a pulverizer, and thus a powder cosmetic base material was produced. This was filled in a middle-sized dish and was compression molded. Thus, a foundation was obtained.

As shown in Table 5, in the foundations of Test Examples 21 to 24 obtained by incorporating spherical porous PMMA particles in an amount of 1% to 20% by mass based on the composition, oily shining was not recognized even after 3 hours, the long-lasting makeup effect was excellent, and usage properties were also maintained. On the other hand, in Test Example 20 produced without incorporating the spherical porous PMMA, oily shining of the skin over time was recognized. Furthermore, in Test Example 25 produced by 30% by mass of the spherical porous PMMA, the long-lasting makeup effect was excellent, but the feeling of use was markedly poor.

**[Table 11]**

| | Test Example | |
|---|---|---|
| | 45 | 46 |
| 1H,1H,2H,2H-perfluorooctyltriethoxysilane | 5 | 5 |
| (5%)/acrylic-silicone copolymer (formula | | |
| (V)) (2%)-treated sericite | | |
| 1H,1H,2H,2H-perfluorooctyltriethoxysilane | Balance | Balance |
| (5%)/acrylic-silicone copolymer (formula | | |
| (V)) (2%)-treated mica | | |
| 1H,1H,2H,2H-perfluorooctyltriethoxysilane | 28 | 28 |
| (5%)/acrylic-silicone copolymer (formula | | |
| (V)) (2%)-treated talc | | |
| 1H,1H,2H,2H-perfluorooctyltriethoxysilane | 12 | 12 |
| (5%)/acrylic-silicone copolymer (formula | | |
| (V)) (2%)-treated titanium oxide fine | | |
| particles | | 4 |
| Aluminum stearate-treated titanium oxide | 4 | |
| fine particles | | |
| 1H, 1H,2H,2H-perfluorooctyltriethoxysilane | 1.2 | 1.2 |
| (5%)/acrylic-silicone copolymer (formula | | |
| (2%)-treated Bengala | | |
| (V)) 1H,1H,2H,2H-perfluorooctyltriethoxysilane | 2.5 | 2.5 |
| (5%)/acrylic-silicone copolymer (formula | | |
| (V)) (2%)-treated yellow iron oxide | | |
| 1H,1H,2H,2H-perfluorooctyltriethoxysilane | 0.9 | 0.9 |
| (5%)/acrylic-silicone copolymer (formula | | |
| black iron oxide | | |
| (V)) (2%)-treated Silicone-based elastomeric powder | 6 | 6 |
| powder | 3 | 3 |
| Spherical polyethylene Spherical porous PMMA | 5 | 5 |
| (average particle size 10 µm, specific | | |
| m²/g) | | |
| surface area 150 | 7 | 7 |
| Fine zinc oxide Fine silica | 3 | 3 |
| | | |
| Paraben | q.s. 3 | q.s. 3 |
| Dimethylpolysiloxane (5 mPa·s) | 3 | 3 |
| Dimethylpolysiloxane (5000 mPa·s) | 2 | 2 |
| Methylphenylpolysiloxane | 3 | 3 |
| Octyl methoxycinnamate Polyether-modified silicone | 1 | 1 |
| Antioxidant | q.s. | q.s. |
| Fragrance | q.s. | q.s. |
| Production method | Preliminarily mixed in | Preliminarily mixed in |
| | Henschel mixer | Henschel mixer |
| | ↓ | ↓ |
| | Mixed two times with facing rotor type mixing | Mixed two times with pulverizier |
| | apparatus | ↓ |
| | ↓ | |
| | Dry press molded in middle-sized resin dish | Dry press molded in middle-sized resin dish |
| Impact resistance | 10 times | 2 times |
| Feeling of use (Fine particulate feeling) | A | B |
| (Moist feeling) | B | C |
| (Smoothness) | A | B |

### (Production method)

Test Example 45: The oily components were added to the powder components of the formulation, and the components were mixed for a certain time in a Henschel mixer (manufactured by Mitsui Miike Machinery Co., Ltd.). Subsequently, the mixture was mixed two times using the facing rotor type mixing apparatus of Fig. 1 (cyclone mill: manufactured by Flo-tec, Ltd.; used by rotating the first rotor and the second rotor in the opposite direction to each other). The mixture was dry press molded in a middle-sized resin dish.
Test Example 46: The oily components were added to the powder components of the formulation, and the components were mixed in a Henschel mixer. Subsequently, the mixture was mixed two times with a pulverizer (manufactured by Hosokawa Micron Corp.), which is a hammer type pulverizing machine, and was dry press molded in a middle-sized resin dish.

As shown in Tables 9 to 11, the foundations of Test Examples 41, 43 and 45 produced using a facing rotor type mixing apparatus had remarkably improved impact resistance as compared with the Test Examples 42, 44 and 46 produced using a pulverizer (hammer type pulverizing machine). Furthermore, it was found that the foundations of Test Examples 41, 43 and 45 were also excellent in the feelings of use such as the feeling of particulate fineness, the moist feeling and smoothness.

The same evaluation was performed with foundations produced by using a facing rotor type mixing apparatus in the same manner as in the above-described test, and varying the amount of incorporation of the spherical polymethyl methacrylate particles (spherical porous PMMA, particle size 10 µm) between 1% to 20% by mass. The results are) presented in the following Table 12.

**[Table 12]**

| | Test Example | | | | |
|---|---|---|---|---|---|
| | 47 | 48 | 49 | 50 | 51 |
| Silicone-treated sericite | 12 | 12 | 12 | 12 | 12 |
| Silicone-treated mica | Balance | Balance | Balance | Balance | Balance |
| Silicone-treated titanium oxide | 23 | 23 | 23 | 23 | 23 |
| Aluminum stearate-treated titanium oxide fine particles | 12 | 12 | 12 | 12 | 12 |
| Silicone-treated Bengala | 4 | 4 | 4 | 4 | 4 |
| Silicone-treated yellow iron oxide | 1.2 | 1.2 | 1.2 | 1.2 | 1.2 |
| Silicone-treated black iron oxide | 2.5 | 2.5 | 2.5 | 2.5 | 2.5 |
| Polyurethane powder | 0.9 | 0.9 | 0.9 | 0.9 | 0.9 |
| Spherical porous PMMA (average particle size 10 µm, specific | 1 | 6 | 10 | 15 | 20 |
| surface area 150 m²/g) | | | | | |
| Fine zinc oxide | 2 | 2 | 2 | 2 | 2 |
| Paraben | q.s. | q.s. | q.s. | q.s. | q.s. |
| Dimethylpolysiloxane (5 mPa·s) | 3 | 3 | 3 | 3 | 3 |
| Hydrogenated polyisobutene (5000 mPa·s) | 2 | 2 | 2 | 2 | 2 |
| Methylphenylpolysiloxane | 3 | 3 | 3 | 3 | 3 |
| Octyl methoxycinnamate | 3 | 3 | 3 | 3 | 3 1 |
| Polyether-modified silicone | 1 | 1 | 1 | 1 | |
| Antioxidant | q.s. | q.s. | q.s. | q.s. | q.s. |
| Fragrance | q.s. | q.s. | q.s. | q.s. | q.s. |
| Production method | Preliminarily mixed in Henschel mixer | | | | |
| | ↓ | | | | |
| | Mixed two times with facing rotor type mixing apparatus | | | | |
| | ↓ | | | | |
| | Dry press molded in middle-sized resin dish | | | | |
| Impact resistance | 20 times | 14 times | 10 times | 7 times | 4 times |
| Feeling of use (Fine particulate feeling) | A | A | A | A | A |
| (Moist feeling) | B | B | B | C | D |
| (Smoothness) | A | A | A | B | B |

(Production method)

## Claims

1. A solid powder cosmetic comprising:
spherical poly(meth)acrylate particles having pores in the interiors and at the surfaces and having an average particle size of 3 to 20 µm, a specific surface area of 80 to 180 m²/g, and a most frequent pore diameter of 180 Å or larger.

2. The solid powder cosmetic according to claim 1, wherein an amount of the spherical poly(meth)acrylate particles is 1% to 20% by mass based on the cosmetic.

3. The solid powder cosmetic according to claim 1 or 2, whrein the the spherical poly(meth)acrylate particles are spherical polymethyl methacrylate (PMMA) particles.

4. The solid powder cosmetic according to any of claim1 to 3, further comprising an activated zinc oxide in an amount of 0.01% to 30% by mass based on the cosmetic.

5. The solid powder cosmetic according to any of claim 1 to 4, further comprising a fluorine compound-treated powder in an amount of 5% to 97% by mass based on the cosmetic.

6. The solid powder cosmetic according to claim 5, wherein the fluorine compound is 1H,1H,2H,2H-perfluorooctyltriethoxysilane.

7. A method for producing a solid powder cosmetic comprising:
mixing of a powder component containing spherical poly(meth)acrylate particles which have pores in the interiors and at the surfaces, and has an average particle size of 3 to 20 µm, a specific surface area of 80 to 180 m²/g, and a most frequent pore diameter of 180Å or larger, with an oily component, by using a facing rotor type mixing apparatus which has a first rotor provided with plural blades and a second rotor provided with plural blades, disposed in a mixing chamber such that the first rotor and the second rotor face each other and respectively have individual rotating shafts on the same axis line in an approximately horizontal direction, and which mixes raw materials by rotating the first rotor and the second rotor in the same or opposite direction to each other, while supplying the raw materials through a feed port on the first rotor side, and discharges the mixed raw materials through a discharge port on the second rotor side.

8. The method for producing a solid powder cosmetic according to claim7, wherein an amount of the spherical poly(meth)acrylate particles is 1% to 10% by mass based on the cosmetic.

9. The method for producing a solid powder cosmetic according to claim7 or 8, whrein the the spherical poly(meth)acrylate particles are spherical polymethyl methacrylate (PMMA) particles.

10. The method for producing a solid powder cosmetic according to any of claim7 to 9, mixing with an activated zinc oxide in an amount of 0.01 % to 30% by mass based on the cosmetic.

11. The method for producing a solid powder cosmetic according to any of claim7 to 10, mixing with a fluorine compound-treated powder in an amount of 5% to 97% by mass based on the cosmetic.

12. The method for producing a solid powder cosmetic according to claim 11, wherein the fluorine compound is 1H,1H,2H,2H-perfluorooctyltriethoxysilane.

13. The method for producing a solid powder cosmetic according to any of claim1 to 12, wherein the first rotor and the second rotor of the facing rotor type mixing apparatus rotate in the opposite direction to each other.
